(19) Europäisches Patentamt
European Patent Office
Office européen des brevets

(11) **EP 4 663 750 A1**

(12) **EUROPEAN PATENT APPLICATION**
published in accordance with Art. 153(4) EPC

(43) Date of publication:
**17.12.2025 Bulletin 2025/51**

(21) Application number: **24753379.7**

(22) Date of filing: **07.02.2024**

(51) International Patent Classification (IPC):
*C12N 5/10* (2006.01)        *A61K 35/15* (2025.01)
*A61P 35/00* (2006.01)       *C12N 15/19* (2006.01)
*C12N 15/28* (2006.01)       *C12N 5/0786* (2010.01)

(52) Cooperative Patent Classification (CPC):
A61K 35/15; A61P 35/00; C07K 14/52;
C07K 14/525; C12N 5/10; C12N 5/06

(86) International application number:
**PCT/JP2024/004080**

(87) International publication number:
**WO 2024/166936 (15.08.2024 Gazette 2024/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.02.2023 JP 2023017913**

(71) Applicant: **Kyushu University, National University Corporation**
**Nishi-ku**
**Fukuoka-shi**
**Fukuoka 819-0395 (JP)**

(72) Inventors:
• **NII, Teruki**
  **Fukuoka-shi, Fukuoka 819-0395 (JP)**
• **TANITO, Kenta**
  **Fukuoka-shi, Fukuoka 819-0395 (JP)**
• **KATAYAMA, Yoshiki**
  **Fukuoka-shi, Fukuoka 819-0395 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

(54) **CELL CAPABLE OF BEING DIFFERENTIATED INTO M2 MACROPHAGE IN TUMOR TISSUE AND HAVING PROPERTY SUCH THAT M2 MACROPHAGE DIFFERENTIATED FROM SAID CELL EXPRESS INFLAMMATORY CYTOKINE, AND COMPOSITION CONTAINING SAID CELL**

(57)    The present disclosure provides a cell that is capable of differentiating into an M2 macrophage within tumor tissue and, upon such differentiation, expresses an inflammatory cytokine, as well as a composition comprising such a cell.

FIG. 16

EP 4 663 750 A1

**Description**

**TECHNICAL FIELD**

The present disclosure relates to a cell that is capable of differentiating into M2 macrophages within tumor tissue and that expresses inflammatory cytokines upon such differentiation, and compositions (e.g., pharmaceutical compositions for use in treating cancer) comprising such cells.

**BACKGROUND ART**

In current cancer treatments using anticancer agents, to achieve a certain therapeutic effect, drugs are generally administered repeatedly (e.g., every two weeks) or at high doses [1-3]. This is due to drug metabolism and reduced efficacy within tumor tissue [4,5]. However, increasing the drug dosage enhances efficacy while simultaneously increasing the risk of side effects [6,7]. Because of such risks and burdens, there is a demand for cancer treatments that do not rely on drugs. Against this backdrop, cancer immunotherapy is considered a promising solution.

However, tumor tissue is generally immunosuppressive, and tumors are not recognized as "foreign" by the immune system [8-13]. Accordingly, cancer immunotherapy has not proven sufficiently effective. This is primarily due to tumor-infiltrating stromal cells such as M2 macrophages (anti-inflammatory) and regulatory T cells that secrete anti-inflammatory cytokines such as interleukin (IL)-10 and transforming growth factor-beta (TGF-$\beta$) [8,14].

Tumor necrosis factor-alpha (TNF-$\alpha$) is one of the most important inflammatory cytokines and plays a major role in the immune activation of surrounding tissues [15,16]. When TNF-$\alpha$ binds to TNF receptor 1 and 2, it activates signaling cascades such as the nuclear factor $\kappa$B (NF-$\kappa$B) pathway, leading to the expression of inflammatory cytokines including IL-6 and IL-1 [16-19]. These inflammatory cytokines induce M1 macrophages (pro-inflammatory) and activate other immune cells, thereby shifting the tissue to an inflammatory environment [15]. Although the inflammation-inducing function of TNF-$\alpha$ is attractive for cancer therapy based on endogenous clearance mechanisms, its severe toxicity even at doses far below therapeutic levels upon systemic administration has hindered its clinical application [20]. Furthermore, since the half-life of TNF-$\alpha$ *in vivo* is approximately 15-30 minutes, delivering a sufficient amount of TNF-$\alpha$ to tumor tissue remains difficult [21,22].

To address this issue, it has recently been reported that TNF-$\alpha$-antibody conjugates can efficiently deliver TNF-$\alpha$ to tumor tissue [23,24]. These technologies enable tumor-specific delivery of TNF-$\alpha$ to antigens expressed on cancer cells. However, if the antigens are mutated or deficient, the efficacy is significantly reduced, indicating room for technical improvement [25,26].

Macrophages are one of the principal immune cells and play a critical role in inflammation and immune responses within tissues [27]. Notably, major features of macrophages in tumor tissue include: (1) macrophages migrate into tumor tissue via C-C chemokine ligand 2 (CCL2) and granulocyte-macrophage colony-stimulating factor (GM-CSF) secreted from cancer cells and stromal cells [28,29]; and (2) macrophages can polarize into the anti-inflammatory M2 phenotype within tumor tissue [18,30,31]. Macrophages switch between M1 and M2 phenotypes depending on the inflammatory status of the tissue environment and regulate the inflammatory milieu accordingly. M2 macrophages are involved in wound healing, tissue regeneration, and immune suppression [30,32]. In particular, in tumor tissue, macrophages become polarized into M2 macrophages and play an essential role in the establishment of an immunosuppressive environment [30].

Furthermore, murine M2 macrophages are known to exhibit very high arginase-1 (Arg1) activity [33]. Arg1 catalyzes the hydrolysis of arginine into urea and ornithine. As a result, the depletion of arginine promotes immunosuppression [34]. Since Arg1 is rarely expressed in M0 or M1 macrophages, Arg1 activity serves as an important marker for identifying the M2 phenotype in mice [35].

SUMMARY **OF THE INVENTION**

The present disclosure provides a cell that is capable of differentiating into M2 macrophages within tumor tissue and that expresses inflammatory cytokines upon differentiation into M2 macrophages, as well as compositions comprising such cells.

According to the present disclosure, for example, the following inventions are provided:

(1) A cell that is capable of differentiating/polarizing into an M2 macrophage, comprising a nucleic acid encoding an inflammatory cytokine operably linked to a regulatory sequence, wherein the cell is capable of expressing the inflammatory cytokine after differentiation/polarization into an M2 macrophage.

(2) The cell according to (1), wherein the cell is selected from the group consisting of M0 macrophages, monocytes, common monocyte progenitors, monocyte-dendritic cell progenitors, myeloid progenitor cells, hematopoietic stem cells, and any cell in the differentiation process from hematopoietic stem cells to M0 macrophages.

(3) The cell according to (1) or (2), wherein the cell is a macrophage that is negative for iNOS, CD206, and Arg1.

(4) The cell according to any one of (1) to (3), wherein the cell is in any stage of differentiation from a monocyte to an M0 macrophage.

(5) The cell according to any one of (1) to (4), wherein the inflammatory cytokine is an inflammatory cytokine selected from the group consisting of TNF-α, IFN-α, IFN-γ, IL-1, IL-2, IL-6, IL-8, IL-12, IL-17, and IL-23.

(6) The cell according to any one of (1) to (5), wherein the inflammatory cytokine is TNF-α.

(7) The cell according to any one of (1) to (6), wherein the regulatory sequence drives expression of the inflammatory cytokine specifically in M2 macrophages.

(8) The cell according to any one of (1) to (7), wherein the regulatory sequence is a regulatory sequence of a gene selected from the group consisting of CD163, CD200R1, CD301, CXCR1, CXCR2, CD209, Dectin-1, FcεRIα, IL-1RII, CD206, arginase-1, IRF4, PPARγ, STAT6, FIZZ1, IL-1ra, IL-10, TGF-β, CCL1, CCL14, CCL18, CCL22, CCL23, CCL24, CCL26, and YM1, or preferably a regulatory sequence of a gene selected from the group consisting of CCL22, LOX, CISH, FCER15, ALOX15, F13A1, IDO1, MMP1, ALDH1A2, CD209, TGM2, VCAM1, MMP12, and SPINT2.

(9) A composition comprising the cell according to any one of (1) to (8).

(10) The composition according to (9), for use in treating cancer.

(11) The composition according to (9), for use in treating cancer in a subject having cancer.

(12) The composition according to (11), wherein the subject is one who has received, is receiving, or is scheduled to receive cancer immunotherapy.

(13) The composition according to (12), wherein the cancer immunotherapy is one or more selected from the group consisting of chimeric antigen receptor-expressing cell therapy, T cell adoptive immunotherapy, and immune checkpoint inhibitor therapy.

(14) The composition according to (11), for use in combination with cancer immunotherapy.

(15) The composition according to any one of (10) to (14), wherein the cancer has an immunosuppressive environment.

(16) The composition according to any one of (10) to (15), wherein the ratio of the number of M1 macrophages to M2 macrophages in the tumor tissue is less than 1.

(17) The composition according to any one of (10) to (16), wherein the subject is resistant to immune checkpoint inhibitor therapy.

(18) The cell according to any one of (1) to (8), wherein the cell is a human cell.

(19) The composition according to any one of (10) to (18), wherein the cell is a human cell.

(20) A method for treating cancer in a subject having cancer, comprising: administering to the subject an effective amount of a cell according to any one of (1) to (8) and (18) to convert at least one immunosuppressive tumor in the subject into a more inflammatory state (e.g., increasing the M1/M2 macrophage ratio in the tumor); and administering to the subject (preferably the subject with the tumor rendered more inflammatory) an effective amount of an immune checkpoint inhibitor.

(21) A composition comprising an effective amount of the cell according to any one of (1) to (8) and (18), for use in the method according to (20).

(22) A composition comprising an effective amount of an immune checkpoint inhibitor, for use in the method according to (20).

(23) A kit comprising an effective amount of the cell according to any one of (1) to (8) and (18) and an effective amount of an immune checkpoint inhibitor, for use in the method according to (20).

(24) A kit comprising a pharmaceutical composition comprising an effective amount of the cell according to any one of (1) to (8) and (18) and a pharmaceutical composition comprising an effective amount of an immune checkpoint inhibitor, for use in the method according to (20).

(25) A pharmaceutical composition comprising an effective amount of the cell according to any one of (1) to (8) and (18) and an effective amount of an immune checkpoint inhibitor, for use in the method according to (20).

(26) Use of the cell according to any one of (1) to (8) and (18) in the manufacture of the composition or kit described above.

(27) Use of a cancer immunotherapeutic agent (e.g., immune checkpoint inhibitor) in the manufacture of the composition or kit described above.

(28) Use of the cell according to any one of (1) to (8) and (18) and a cancer immunotherapeutic agent (e.g., immune checkpoint inhibitor) in the manufacture of the composition or kit described above.

**BRIEF DESCRIPTION OF THE DRAWINGS**

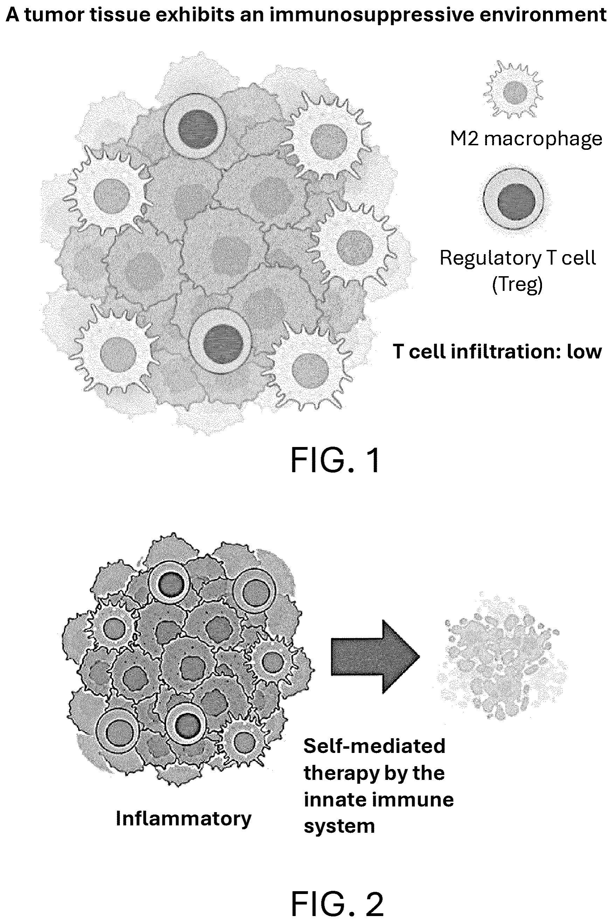

FIG. 1 shows that tumor tissue exhibits an immunosuppressive environment, where regulatory T cells and M2 macrophages are present, and T cell infiltration is low.

Figure 2 shows that, when a tumor tissue is placed under an inflammatory environment, the tumor tissue can shrink or disappear due to the effect of immunity inherently possessed by the individual.

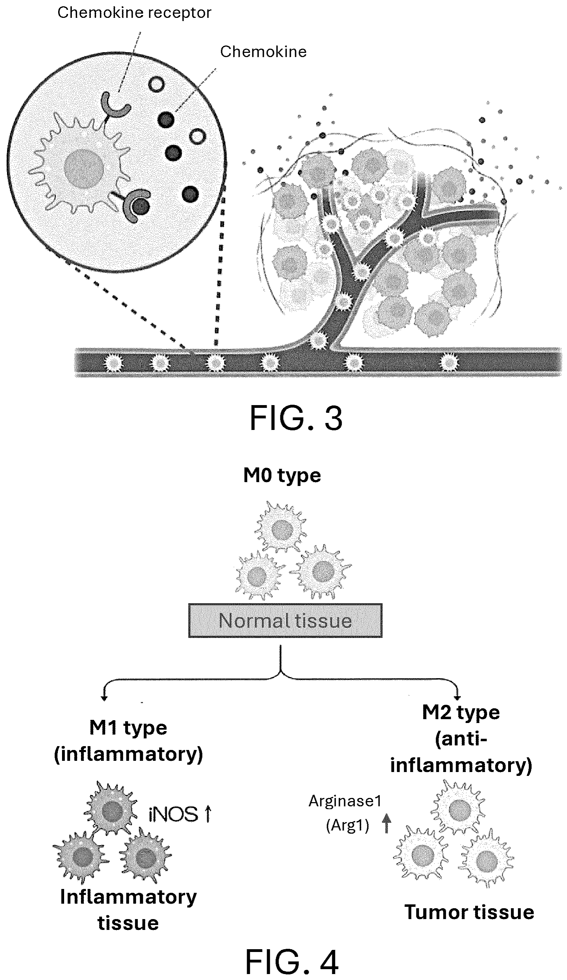

FIG. 3 shows that macrophages migrate into tumor tissue and polarize into M2 macrophages within the tumor.

FIG. 4 shows that macrophages in normal tissue are in the M0 state, but polarize into the M2 phenotype within tumor tissue.

FIG. 5A shows a scheme in which a nucleic acid comprising a gene encoding an inflammatory cytokine (e.g., TNF-$\alpha$) under the control of an M2-specific promoter is introduced into M0 macrophages, which are then induced to M2 macrophages to evaluate cytokine (e.g., TNF-$\alpha$) expression.

FIG. 5B shows that M0 macrophages treated with IL-4 or IL-13, which are M2-inducing cytokines, differentiate into M2 macrophages and produce TNF-$\alpha$ in an M2-specific manner, and similarly, MO macrophages treated with tumor-conditioned medium (TCM) differentiate into M2 macrophages and produce TNF-$\alpha$ specifically in M2.

FIG. 5C shows that M0 macrophages treated with IL-4 or IL-13 differentiate into M2 macrophages and produce cytokines (IL-2 and IL-12) derived from transgenes linked to M2-specific promoters in an M2-specific manner.

FIG. 6A shows that M1 markers are upregulated in tumor tissue of the MacTrigger-treated group.

FIG. 6B shows an increased M1/M2 macrophage ratio in the tumor tissue of the MacTrigger-treated group.

FIG. 6C shows that MacTrigger polarizes into M2 macrophages in tumor tissue and secretes TNF-$\alpha$ within the tumor.

FIG. 7A shows a scheme for measuring tumor size in tumor-bearing mice treated with MacTrigger.

FIG. 7B shows time-dependent changes in tumor size and survival curves in the MacTrigger-treated group.

FIG. 7C shows the results of an in vitro experiment illustrating the transition of cell viability of MacTrigger cultured in the presence of M2-inducing cytokines or in the presence of TCM.

FIG. 7D shows the expression of the proliferation marker Ki67 in tumor tissue of the MacTrigger-treated group.

FIG. 7E shows the transition of body weight in the MacTrigger-treated group.

FIG. 8A shows a scheme for determining the proportions of NK cells and cytotoxic T cells in the tumor tissue of the MacTrigger-treated group.

FIG. 8B shows the proportions of NK cells and cytotoxic T cells in the tumor tissue of the MacTrigger-treated group.

FIG. 9A shows the dynamics of VT-680-labeled macrophages in the body of mice after administration of VT-680-labeled macrophages.

FIG. 9B shows the amount of VT-680-labeled macrophages present in non-tumor tissue and tumor tissue of mice after administration of VT-680-labeled macrophages.

FIG. 9C shows the amount of VT-680-labeled macrophages present in each tissue of mice after administration of VT-680-labeled macrophages.

FIG. 10A shows the analysis scheme for cells in tumor tissue and liver in the macrophage-treated group.

FIG. 10B shows AST activity in mice administered with macrophages.

FIG. 10C shows the expression levels of CD206 (an M2 marker) in tumor tissue and liver of the macrophage-treated group.

FIG. 10D shows the presence or absence of hepatomegaly and the blood AST levels in the MacTrigger-treated group.

FIG. 10E shows the degree of immune cell infiltration in the liver of the MacTrigger-treated group.

FIG. 11A shows the effect of MacTrigger administration on larger tumors at 10 days after tumor implantation. The left panel shows the time-dependent change in tumor volume, and the right panel shows the M1 macrophage / M2 macrophage ratio in the tumor.

FIG. 11B shows the effect of MacTrigger in mice grafted with 4T1 or Colon-26 cells. The graph shows time-dependent changes in tumor volume.

FIG. 12A shows shows the combinatorial effect of MacTrigger and anti-PD-1 antibody in tumor-bearing mice grafted with tumor cells resistant to immune checkpoint inhibitors. Figure 12A shows the time-dependent change in tumor volume after MacTrigger administration.

FIG. 12B shows changes in the proportion of PD-1-positive cells among CD4 single-positive T cells after MacTrigger treatment.

FIG. 12C shows the change in body weight after combination treatment of MacTrigger and anti-PD-1 antibody in tumor-bearing mice grafted with tumor cells resistant to immune checkpoint inhibitors.

FIG. 12D shows the effect of combination treatment with MacTrigger and anti-PD-1 antibody on liver weight (left) and spleen weight (right) in tumor-bearing mice grafted with tumor cells resistant to immune checkpoint inhibitors.

FIG. 13 shows the effect of MacTrigger administration, and the combination of MacTrigger and anti-PD-1 antibody, in mice bearing triple-negative breast cancer cells. Figure 13 shows the time-dependent change in tumor volume after administration.

FIG. 14A shows the expression ratio of genes in human macrophages (expression level in M2 / expression level in M0); **** indicates $p < 0.001$.

FIG. 14B shows the gene expression ratio (expression level in M2 / expression level in M1) in human macrophages; **** indicates p < 0.001.

FIG. 14C shows the gene expression ratio (expression level in M2 / expression level in M0) in human macrophages; **** indicates p < 0.001.

FIG. 15 shows M2 macrophage-specific cytokine production by human MacTrigger.

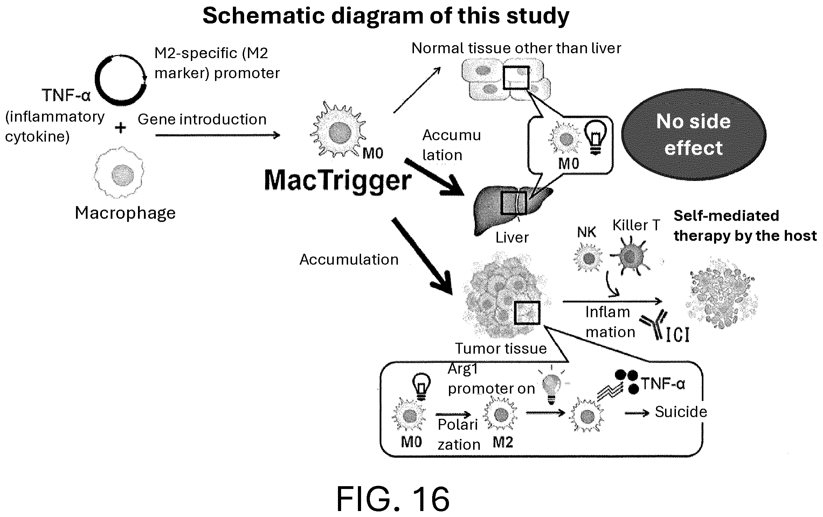

FIG. 16 illustrates an overview of the cancer treatment strategy described in the present disclosure.

## DETAILED DESCRIPTION OF THE INVENTION

In the present invention, the term "subject" refers to a vertebrate, including, for example, birds and mammals. Exemplary mammals include mice, rats, hamsters, guinea pigs, horses, cattle, pigs, goats, sheep, donkeys, dogs, and cats, as well as primates such as monkeys, chimpanzees, gorillas, orangutans, bonobos, humans, and particularly humans. In this specification, the term "subject" is used to include humans unless explicitly excluded, in which case the term "non-human" is used.

As used in this specification, the term "treatment" may encompass both prophylactic and therapeutic treatments. Prophylactic treatment includes preventing the onset of a disease, delaying its onset, or reducing its incidence. Therapeutic treatment includes slowing the progression of a disease, delaying its worsening, preventing further deterioration, alleviating symptoms, curing the disease, and achieving remission.

As used in this specification, "macrophage" refers to a type of leukocyte that migrates within the body via amoeboid movement. Macrophages exhibit phagocytic activity and can engulf foreign substances such as bacteria, viruses, and dead cells. Macrophages are key immune cells that play a pivotal role in tissue inflammation and immune responses [27]. Notably, in tumor tissue, macrophages exhibit major characteristics: (1) macrophages migrate into tumor tissue via C-C chemokine ligand 2 (CCL2) and granulocyte-macrophage colony-stimulating factor (GM-CSF), which are secreted from cancer cells and stromal cells [28,29]; and (2) in tumor tissue, macrophages can be polarized into the anti-inflammatory M2 phenotype [18,30,31]. Macrophages change into M1 or M2 phenotypes depending on the inflammatory state of the tissue microenvironment and regulate the inflammatory milieu. M2 macrophages are involved in wound healing, tissue regeneration, and immunosuppression [30,32]. Particularly in tumor tissue, macrophages polarize into M2 macrophages and play a key role in establishing the immunosuppressive environment [30]. Furthermore, mouse M2 macrophages are known to have very high arginase 1 (Arg1) activity [33]. Arg1 catalyzes the hydrolysis of arginine into urea and ornithine. As a result, the amount of arginine decreases, which promotes immunosuppression [34]. Since Arg1 is not significantly expressed in M0 or M1 macrophages, Arg1 activity serves as an important marker for identifying the mouse M2 phenotype [35]. M1 macrophages express one or more, or all, of the molecules selected from the group consisting of CD80, CD86, TLR-2, TLR-4, iNOS, and MHC-II, and preferably secrete one or more, or all, of the cytokines selected from the group consisting of interferon-$\alpha$ (TNF-$\alpha$), interleukin-1$\alpha$ (IL-1$\alpha$), IL-1$\beta$, IL-6, IL-12, IL-23, CXCL9, CXCL16, and CCL5. M2 macrophages express one or more, or all, of the molecules selected from the group consisting of CD163, CD206, CD209, FIZZ1, and Ym1/2, and preferably secrete one or more, or all, of the cytokines selected from the group consisting of IL-10, TGF-$\beta$, CCL1, CCL17, CCL18, CCL22, CCL24, CXCL13, and VEGF. M1 macrophages are induced, for example, from monocytes by LPS, granulocyte-macrophage colony-stimulating factor (GM-CSF), IFN-y, or TNF-$\alpha$. M2 macrophages are induced, for example, from monocytes by IL-4, IL-13, IL-10, or IL-21. M2 macrophages are known to include four subtypes: M2a, M2b, M2c, and M2d. As markers for human M2 macrophages, the following are listed: IDO, IL-10, TGF-$\beta$, CD115, CD204, CD163, CD206, CD209, Fc$\epsilon$RI, VSIG4, IRF4, and STAT6. Therefore, human M2 macrophages can be identified by one or more, two or more, three or more, four or more, five or more, six or more, seven or more, eight or more, nine or more, ten or more, eleven or more, or all twelve of the group consisting of IDO, IL-10, TGF-$\beta$, CD115, CD204, CD163, CD206, CD209, Fc$\epsilon$RI, VSIG4, IRF4, and STAT6. Naive macrophages (macrophages that have not been stimulated) are referred to as M0 macrophages. M0 macrophages reversibly polarize into M1 and M2 macrophages. M0 macrophages are differentiated from monocytes. Monocytes differentiate from hematopoietic stem cells through myeloid progenitor cells, monocyte-dendritic progenitor cells, and common monocyte progenitor cells. MO macrophages can be obtained by stimulating monocytes with phorbol 12-myristate 13-acetate (PMA) or macrophage colony-stimulating factor (M-CSF). MO macrophages can be characterized by being negative for M1 macrophage markers and M2 macrophage markers.

As used in this specification, the term "regulatory sequence" refers to a sequence that drives a gene operably linked thereto and has the activity of transcribing RNA from the gene. The regulatory sequence may be, for example, a promoter. Promoters include, for example, class I promoters (which may be used for transcription of rRNA precursors), class II promoters (which consist of a core promoter and upstream promoter elements and may be used for mRNA transcription), and class III promoters (which can be further classified into type I, II, and III). The term "control sequence," also referred to as a regulatory sequence, and may be any promoter capable of transcribing mRNA within cells such as animal or plant cells. As the first control sequence, various RNA pol II promoters may be employed. Examples of suitable pol II promoters include, but are not limited to, the CMV promoter, EF 1 promoter (EF 1$\alpha$ promoter), SV40 promoter, MSCV promoter, hTERT promoter, β-actin promoter, CAG promoter, and CBh promoter. Additionally, the term "promoter" herein also includes promoters that drive bacteriophage-derived RNA polymerases such as the T7 promoter, T3 promoter, and SP6 promoter, as well as pol III promoters such as the U6 promoter. The T7 promoter is preferably used for transcription from circular DNA, while the SP6 promoter is preferably used for transcription from linear DNA. The promoter may also be an inducible promoter. An inducible promoter refers to a promoter that drives expression of a polynucleotide operably linked to the promoter only in the presence of an inducer that drives the promoter. Some inducible promoters may function such that they induce expression of the operably linked polynucleotide only in the absence of an inhibitor that suppresses promoter activity. Inducible promoters include, for example, heat shock promoters that induce gene expression upon heat stimulation, although they are not limited thereto. Drug-inducible promoters are also encompassed by the inducible promoters. Examples of such drug-inducible promoters include cumate operator sequences, lambda operator sequences (e.g., 12× lambda Op), and tetracycline-inducible promoters. Tetracycline-inducible promoters include those that drive gene expression in the presence of tetracycline, a tetracycline derivative such as doxycycline, or a reverse tetracycline-controlled transactivator (rtTA). An example of a tetracycline-inducible promoter is the TRE3G promoter. The control sequence is capable of driving expression of a gene operably linked thereto at least in M2 macrophages. The term "operably linked" means that the gene is connected to the control sequence in such a manner that its expression is regulated by the control sequence.

As used herein, the term "cell" refers to a vertebrate cell, preferably a mammalian cell, and more preferably a human cell. A cell having the ability to differentiate or polarize into an M2 macrophage refers to a hematopoietic stem cell, myeloid progenitor cell, monocyte-dendritic progenitor cell, common monocyte progenitor cell, monocyte, or MO macrophage, or any cell within the differentiation pathway thereof. Each of these cell types, when administered to a subject, can differentiate into an M2 macrophage, at least in part, within the body of the subject. In particular, hematopoietic stem cells differentiate into monocytes in the bone marrow via myeloid progenitor cells, monocyte-dendritic progenitor cells, and common monocyte progenitor cells. The differentiated monocytes further differentiate into macrophages in peripheral tissues. Macrophages migrate to tumor tissues and polarize into M2 macrophages.

As used in this specification, the term "potential for differentiation/polarization" means either a potential for differentiation or a potential for polarization, or both. The term "differentiation/polarization" refers to either differentiation or polarization, or both. Polarization is used to describe a reversible change among M0, M1, and M2 states. Differentiation is used to describe changes in cellular stages other than polarization.

According to the present disclosure, there is provided a cell having a potential for differentiation/polarization into M2 macrophages. In a preferred embodiment, the cell having such potential comprises a nucleic acid, more preferably a nucleic acid encoding an inflammatory cytokine, operably linked to a regulatory sequence. According to the present disclosure, there is provided a cell comprising a nucleic acid encoding an inflammatory cytokine operably linked to a regulatory sequence. In one preferred embodiment, such a cell is capable of expressing the inflammatory cytokine after differentiation/polarization into an M2 macrophage.

According to the present disclosure, there is provided a cell having a potential for differentiation/polarization into an M2 macrophage, comprising a nucleic acid encoding an inflammatory cytokine operably linked to a regulatory sequence, and capable of expressing the inflammatory cytokine after differentiation/polarization into the M2 macrophage. Such a cell can differentiate or polarize into an M2 macrophage within tumor tissue, thereby expressing the inflammatory cytokine in the tumor tissue. As a result, the immunosuppressive state of the tumor tissue can be alleviated, and preferably, the tumor microenvironment can be converted into an inflammatory state (or an environment with a ratio of M1 macrophages to M2 macrophages (M1/M2) > 1). By converting the tumor tissue into an inflammatory environment, natural killer (NK) cells or T cells can be recruited to the tumor site, thereby eliciting or enhancing an antitumor effect. The antitumor effect in the tumor tissue of a subject receiving the cell of the present disclosure is preferably achieved primarily through activation of the subject's immune system other than the administered cells, specifically via recruitment and activation of immune cells such as NK cells or T cells present in the subject.

In one preferred embodiment, the cell having the potential for differentiation/polarization into M2 macrophages is a cell selected from the group consisting of M0 macrophages, monocytes, common monocyte progenitor cells, monocyte-dendritic progenitor cells, myeloid progenitor cells, and hematopoietic stem cells, and any cell in the differentiation process from a hematopoietic stem cell to an M0 macrophage. In one preferred embodiment, the cell having a potential for differentiation/polarization into M2 macrophages may be a cell at a differentiation stage between a hematopoietic stem cell and a myeloid progenitor cell. In one preferred embodiment, the cell having a potential for differentiation/polarization into M2 macrophages may be a cell at a differentiation stage between a myeloid progenitor cell and a monocyte-dendritic progenitor cell. In one preferred embodiment, the cell having a potential for differentiation/polarization into M2 macrophages may be a cell at a differentiation stage between a monocyte-dendritic progenitor cell and a common monocyte progenitor cell. In one preferred embodiment, the cell having a potential for differentiation/polarization into M2 macrophages may be a cell at a differentiation stage between a common monocyte progenitor cell and a monocyte. In one preferred embodiment, the cell having a potential for differentiation/polarization into M2 macrophages may be a cell at a differentiation stage between a monocyte and an M0 macrophage. In a more preferred embodiment, the cell having a potential for differentiation/polarization into M2 macrophages is any cell in the differentiation process from a monocyte to an MO macrophage, and even more preferably, is an M0 macrophage. In one preferred embodiment, the cell having a potential for differentiation/polarization into M2 macrophages may be a macrophage that is negative for iNOS, CD206, and Arg-1 (that is, a macrophage that is neither an M1 macrophage nor an M2 macrophage).

In one embodiment, the inflammatory cytokine can alleviate (reduce) the immunosuppressive state of the tumor tissue. In one preferred embodiment, the inflammatory cytokine can convert the tumor tissue from an immunosuppressive environment to an inflammatory environment. In one preferred embodiment, the inflammatory cytokine is an inflammatory cytokine selected from the group consisting of tumor necrosis factor-alpha (TNF-$\alpha$), IFN-$\gamma$, IL-1 (for example, IL-1$\beta$), IL-2, IL-6, IL-12, IL-17, and IL-23. In one preferred embodiment, the inflammatory cytokine is an inflammatory cytokine selected from the group consisting of tumor necrosis factor-alpha (TNF-$\alpha$), IFN-$\alpha$, IFN-$\gamma$, IL-1 (for example, IL-1$\beta$), IL-2, IL-6, IL-8, IL-12, IL-17, and IL-23. In one preferred embodiment, the inflammatory cytokine may be TNF-$\alpha$. Human TNF-$\alpha$ may, for example, have the sequence registered under GenBank: QCI55793.1, or a TNF-$\alpha$ sequence corresponding to said sequence. Human TNF-$\alpha$ may include, for example, peptides having 90% or more sequence identity to the sequence registered under GenBank: QCI55793.1 and having the function of human TNF-$\alpha$. The function of human TNF-$\alpha$ may be the function of inducing inflammation in a human subject. When administered to a human, the inflammatory cytokine is not particularly limited as long as it is a cytokine capable of inducing inflammation in humans, but is preferably a human cytokine.

In one preferred embodiment, the regulatory sequence can express a gene, such as an inflammatory cytokine, operably linked to said regulatory sequence in M2 macrophages. In one preferred embodiment, the regulatory sequence can express a gene, such as an inflammatory cytokine, operably linked thereto selectively or specifically in M2 macrophages. Here, "selectively" or "specifically" in M2 macrophages means that the regulatory sequence drives stronger gene expression in M2 macrophages than in at least M0 macrophages and M1 macrophages (2-fold or more, 3-fold or more, 4-fold or more, 5-fold or more, 6-fold or more, 7-fold or more, 8-fold or more, 9-fold or more, 10-fold or more, 20-fold or more, 30-fold or more, 40-fold or more, 50-fold or more, 100-fold or more, 500-fold or more, or 1000-fold or more stronger gene expression). In one embodiment, the regulatory sequence is a regulatory sequence of a gene selected from the group consisting of CD163, CD200R1, CD301, CXCR1, CXCR2, CD209, Dectin-1, Fc$\epsilon$RI$\alpha$, IL-1RII, CD206, arginase-1, IRF4, PPAR$\gamma$, STAT6, FIZZ1, IL-1ra, IL-10, TGF-$\beta$, CCL1, CCL14, CCL18, CCL22, CCL23, CCL24, CCL26, and YM1. In one embodiment, the regulatory sequence may be a promoter of a gene selected from the group consisting of CCL-22, LOX, CISH, FCER15, ALOX15, F13A1, IDO1, MMP1, ALDH1A2, CD209, TGM2, VCAM1, MMP12, and SPINT2. In one preferred embodiment, the regulatory sequence may be a promoter of a gene selected from the group consisting of CCL-22, CISH, ALOX15, and TGM2. The regulatory sequence is preferably derived from the human genome. The regulatory sequence is preferably a promoter of a human gene selected from the group consisting of CCL-22, CISH, ALOX15, and TGM2. In one embodiment, the regulatory sequence may be CCL-22. In one embodiment, the regulatory sequence may be CD209.

In one preferred embodiment, according to the present disclosure, there is provided a cell that is in any differentiation stage from a monocyte to an M0 macrophage, comprising a nucleic acid encoding an inflammatory cytokine operably linked to a regulatory sequence that is selective or specific for M2 macrophages, and capable of expressing said inflammatory cytokine after differentiation/polarization into an M2 macrophage. In one preferred embodiment, according to the present disclosure, there is provided a cell that is in any differentiation stage from a monocyte to an M0 macrophage, comprising a nucleic acid encoding TNF-$\alpha$ operably linked to a regulatory sequence that is selective or specific for M2 macrophages, and capable of expressing said TNF-$\alpha$ after differentiation/polarization into an M2 macrophage. In one preferred embodiment, the cell is a monocyte or M0 macrophage. In one preferred embodiment, the cell is a macrophage that is non-M1 nor non-M2. In one preferred embodiment, the cell is, for example, a macrophage that is negative for iNOS, CD206, and Arg-1.

In one preferred embodiment, according to the present disclosure, there is provided an M0 macrophage comprising a nucleic acid encoding an inflammatory cytokine operably linked to a regulatory sequence that is selective or specific for M2 macrophages, and capable of expressing said inflammatory cytokine after differentiation/polarization into an M2 macrophage. In one preferred embodiment, according to the present disclosure, there is provided an M0 macrophage comprising a nucleic acid encoding TNF-$\alpha$ operably linked to a regulatory sequence that is selective or specific for M2 macrophages, and capable of expressing said TNF-$\alpha$ after differentiation/polarization into an M2 macrophage.

In one embodiment, the cell of the present disclosure releases an inflammatory cytokine in tumor tissue. In one embodiment, the cell of the present disclosure increases the M1 macrophage number/M2 macrophage number ratio (M1 number /M2 number ratio) to 1 or more in tumor tissue. In one embodiment, the cell of the present disclosure converts the tumor tissue to an inflammatory environment. In one embodiment, the cell of the present disclosure increases immune cells such as NK cells and/or T cells in tumor tissue.

According to the present disclosure, there is provided a composition comprising the cell of the present disclosure. The composition of the present disclosure may further comprise a pharmaceutically acceptable solution in addition to said cell. The composition of the present disclosure may further comprise a pharmaceutically acceptable additive (for example, pharmaceutical excipients such as salts, buffers, thickeners, isotonic agents, dispersants) in addition to said cell. The composition of the present disclosure may further comprise a pharmaceutically acceptable solution and a pharmaceutically acceptable additive (for example, salts, buffers, thickeners, isotonic agents, dispersants) in addition to said cell.

According to the present disclosure, there is provided a composition comprising the cell of the present disclosure for use in releasing an inflammatory cytokine in tumor tissue. According to the present disclosure, there is provided a composition comprising the cell of the present disclosure for use in converting tumor tissue into an inflammatory environment. According to the present disclosure, there is provided a composition comprising the cell of the present disclosure for use in increasing the M1 macrophage number/M2 macrophage number in tumor tissue to 1 or more. According to the present disclosure, there is provided a composition comprising the cell of the present disclosure for use in increasing immune cells such as NK cells and/or T cells in tumor tissue.

In one embodiment, the composition of the present disclosure can be used for treating cancer. In one embodiment, the composition of the present disclosure can be used for treating a cancer patient. In one embodiment, the composition of the present disclosure can be used for alleviating the immunosuppressive state of cancer tissue in a cancer patient. In one embodiment, the composition of the present disclosure can be used for changing the immunosuppressive state of cancer tissue in a cancer patient to an inflammatory state. In one embodiment, the composition of the present disclosure can be used for recruiting immune cells into cancer tissue in a cancer patient. In one embodiment, the composition of the present disclosure can be used for treating cancer (for example, therapeutically treating) in a cancer patient.

The cancer is not particularly limited, but includes, for example, hematologic cancers such as acute lymphoblastic leukemia (ALL), acute myeloid leukemia (AML), Hodgkin lymphoma, non-Hodgkin lymphoma, B-cell lymphoma, multiple myeloma, and T-cell lymphoma; solid tumors such as melanoma, myelodysplastic syndrome, adenocarcinoma, squamous cell carcinoma, adenosquamous carcinoma, undifferentiated carcinoma, large cell carcinoma, non-small cell lung cancer, small cell lung cancer, mesothelioma, skin cancer, breast cancer (including triple-negative breast cancer), prostate cancer, bladder cancer, vaginal cancer, cervical cancer, head and neck cancer, uterine cancer, cervical cancer, liver cancer, gallbladder cancer, bile duct cancer, kidney cancer, pancreatic cancer, lung cancer, colon cancer, colorectal cancer, rectal cancer, small intestinal cancer, gastric cancer, esophageal cancer, testicular cancer, ovarian cancer, bladder cancer, brain tumors, and cancers of bone tissue, cartilage tissue, adipose tissue, muscle tissue, vascular tissue, and hematopoietic tissue; sarcomas such as chondrosarcoma, Ewing sarcoma, malignant hemangioendothelioma, malignant schwannoma, osteosarcoma, and soft tissue sarcoma; and blastomas such as hepatoblastoma, medulloblastoma, nephroblastoma, neuroblastoma, pancreatoblastoma, pleuropulmonary blastoma, and retinoblastoma.

According to the present disclosure, the composition or cell of the present disclosure can be administered parenterally to a subject. Parenteral administration is not particularly limited, but includes, for example, intravenous administration, intra-arterial administration, intramuscular administration, intradermal administration, subcutaneous administration, intraperitoneal administration, intrathoracic administration, intra-articular administration, intramyocardial administration, intraventricular administration, intracerebroventricular administration, intramedullary injection, and cerebrospinal fluid administration. In one preferred embodiment, the parenteral administration may be intravenous administration.

According to the present disclosure, there is provided a method for treating cancer in a subject in need thereof, comprising administering to said subject a therapeutically effective amount of the cells of the present disclosure. In one embodiment, the cancer tissue of said subject transitions to an inflammatory environment.

According to the present disclosure, there is provided a method for treating a subject having cancer, comprising administering to said subject a therapeutically effective amount of the cells of the present disclosure. In one embodiment, the cancer tissue of said subject transitions to an inflammatory environment.

According to the present disclosure, there is provided a method for producing inflammatory cytokines in tumor tissue of a subject having cancer, the method comprising administering to the subject an effective amount of the cell of the present disclosure. According to the present disclosure, there is provided a method for alleviating the immunosuppressive state of tumor tissue in a subject having cancer, the method comprising administering to the subject an effective amount of the cell of the present disclosure. According to the present disclosure, there is provided a method for converting tumor tissue of a subject having cancer (for example, from an immunosuppressive state) to an inflammatory environment, the method comprising administering to the subject an effective amount of the cell of the present disclosure. According to the present disclosure, there is provided a method for increasing the M1 macrophage number / M2 macrophage number in tumor tissue of a subject having cancer to 1 or more, the method comprising administering to the subject an effective amount of the cell of the present disclosure.

According to the present disclosure, the subject having cancer may be a subject before, during, or after immunotherapy. According to the present disclosure, the subject having cancer may be treated by a combination of administration of the cell of the present disclosure and a cancer therapy. According to the present disclosure, the subject having cancer receives administration of an effective amount of the cell of the present disclosure and a cancer therapy.

Administration of an effective amount of the cell of the present disclosure to a subject having cancer ensures that the tumor becomes an inflammatory tumor, and an inflammatory tumor may elicit a response from the immune system in the body and initiate an attack on the tumor. Therefore, administration of an effective amount of the cell of the present disclosure to a subject having cancer may make any combination with cancer immunotherapy or antibody therapy more effective. The cancer therapy may be, for example, antibody therapy. In antibody therapy, an antibody that binds to a cancer antigen and has antibody-dependent cellular cytotoxicity (ADCC activity) or complement-dependent cytotoxicity (CDC activity) can be administered to a cancer patient. Administration of the cell of the present disclosure can convert a non-inflammatory tumor to an inflammatory state, thereby promoting the recruitment of NK cells into tumor tissue, and thereby enabling the antibody bound to cancer cells within the tumor tissue to exert ADCC activity. The antibody may bind to the cancer antigen, for example, with a binding dissociation constant (KD) of $10^{-7}$ M or less, $10^{-8}$ M or less, $10^{-9}$ M or less, $10^{-10}$ M or less, or $10^{-11}$ M or less, or within the range of $10^{-8}$ M to $10^{-11}$ M. The antibody preferably has high binding selectivity to cancer cells and desirably does not show significant binding to normal cells. The cancer immunotherapy may be, for example, T cell adoptive immunotherapy, chimeric antigen receptor (CAR)-expressing immune cell therapy, or immune checkpoint inhibitor therapy. The T cell adoptive immunotherapy comprises administering to a subject having cancer an effective amount of T cells that bind to a cancer antigen. The T cells may be those expanded by cell culture, or may be rejuvenated T cells that have undergone reprogramming to iPS cells; for example, T cells binding to a cancer antigen may be collected from a cancer patient, reprogrammed to generate iPS cells, and T cells obtained by differentiation induction from the iPS cells may be used (see, for example, US 9,206,3934 B). The T cells may be T cells having a homogeneous T cell receptor (TCR) by cloning the iPS cells and/or by suppressing or stopping expression of RAG1 and RAG2 (see, for example, US 9,206,3934 B). The chimeric antigen receptor (CAR)-expressing immune cell therapy comprises administering to a subject having cancer an effective amount of CAR immune cells (for example, CAR-expressing T cells and CAR-expressing NK cells, etc.) that bind to a cancer antigen. The immune checkpoint inhibitor therapy comprises administering to a subject having cancer an effective amount of an immune checkpoint inhibitor. Immune checkpoint inhibitors release immune suppression, that is, release the brakes; however, they are considered to have low efficacy when the immune system is not activated in the first place. Administration of an effective amount of the cells of the present disclosure can enhance tumor inflammation and activate immunity against the tumor, and therefore is expected to improve the efficacy of immune checkpoint inhibitors.

According to the present disclosure, the cell of the present disclosure or a cancer immunotherapeutic agent, or a pharmaceutical composition comprising the same, for use in a method for treating cancer comprising administration of an effective amount of the cell of the present disclosure and cancer immunotherapy, is provided. According to the present disclosure, use of the cell of the present disclosure or a cancer immunotherapeutic agent, or both, in the manufacture of a medicament for use in a method for treating cancer comprising administration of an effective amount of the cell of the present disclosure and cancer immunotherapy, is provided.

The antibody is not particularly limited, but includes, for example, rituximab, trastuzumab, cetuximab, alemtuzumab, ofatumumab, obinutuzumab, daratumumab, elotuzumab, and avelumab. In antibody therapy, humanized or human chimeric IgG1 antibodies that bind to cancer antigens may also be administered.

Examples of CAR-expressing cells include CAR-expressing immune cells. The immune cells are not particularly limited, and may include, for example, T cells, NK cells, neutrophils, and macrophages. The "chimeric antigen receptor" (CAR) is a chimeric molecule comprising an antigen-binding fragment of an antibody that binds to a cancer antigen (particularly, scFv) and an activation domain of an immune cell. A CAR is generally a molecule in which a scFv, an extracellular hinge domain, a transmembrane domain (e.g., CD8$\alpha$ or CD28), and an activation signaling domain (e.g., CD3$\zeta$) are linked. A CAR can be introduced into a cell and expressed on the cell surface. A cell expressing a CAR can be targeted to a specific antigen. For example, CAR can be introduced into an immune cell such as a T cell or NK cell to target the immune cell such as a T cell or NK cell to cancer. In a first-generation CAR, an scFv, an extracellular hinge domain, a transmembrane domain (e.g., CD8$\alpha$ or CD28), and an activation signaling domain (e.g., CD3$\zeta$) are connected. A second-generation CAR further comprises a costimulatory signaling domain for activating immune cells into which the CAR has been introduced. Examples of costimulatory signaling domains include costimulatory molecules such as CD28, 4-1BB, OX40, CD27, and ICOS. A third-generation CAR incorporates multiple costimulatory molecules. In this way, improvements have been made in CARs to enable immune cells into which CARs are introduced to proliferate persistently in vivo. It is preferable that domains other than the scFv portion be derived from human proteins.

The antigen of the antibody or the antigen-binding fragment of the antibody (e.g., scFv) may be an antigen expressed on the surface of a cancer cell (e.g., a cancer antigen). The cancer antigen may include, for example, one or more selected from the group consisting of CD16, CD19, CD20, CD22, CD123, CD171, epidermal growth factor receptor (EGFR), particularly EGFRvIII, type III EGFR, de2-7EGFR, and HER2, carcinoembryonic antigen (CEA), prostate stem cell antigen (PSCA), B-cell maturation antigen (BCMA), CS1, NKG2D, NKp30, B7H6, MUC-16 (CA125), receptor tyrosine kinase-like orphan receptor 1 (ROR-1), GD3, GM2, glypican-3 (GPC3), mesothelin, IL13R, c-KIT, c-MET, NY-ESO-1, WT1, MAGE-A3, MAGE-A4, MAGE-A10, HPV E6, HPV E7, CMV, AFP, PRAME, SSX2, KRAS, HER2, and PD-L1. The antibody or the antigen-binding fragment of the antibody (e.g., scFv) may also be a protein tag, for example. The antibody or antigen-binding fragment of the antibody (e.g., scFv) may be, for example, a fluorescent protein such as fluorescein isothiocyanate (FITC).

Examples of CAR-T cell therapies approved by the U.S. Food and Drug Administration (FDA) include: Abecma (generic name: idecabtagene vicleucel), Breyanzi (generic name: lisocabtagene maraleucel), Carvykti (generic name: ciltacabtagene autoleucel), Kymriah (generic name: tisagenlecleucel), Tecartus (generic name: brexucabtagene autoleucel), and Yescarta (generic name: axicabtagene ciloleucel). Other CAR-T cell therapies in addition to these may also be favorably combined with the administration of the cells of the present disclosure.

Immune checkpoint inhibitors (ICIs) can inhibit the function of immune checkpoints, for example, by binding to immune checkpoint molecules or their ligands. Immune checkpoint molecules include programmed cell death-1 (PD-1), cytotoxic T-lymphocyte-associated protein 4 (CTLA-4), T-cell immunoglobulin domain and mucin domain-3 (TIM-3), lymphocyte activation gene 3 (LAG-3), and V-type immunoglobulin domain-containing suppressor of T-cell activation (VISTA), as well as signal regulatory protein alpha (SIRPα), which is dendritic cell-specific. The immune checkpoints mediated by each of these are referred to as PD-1 axis immune checkpoint, CTLA-4 axis immune checkpoint, TIM-3 axis immune checkpoint, LAG-3 axis immune checkpoint, VISTA axis immune checkpoint, and SIRPα axis immune checkpoint, respectively. For example, by inhibiting the binding between PD-1 and PD-L1 or PD-L2, the PD-1 axis immune checkpoint can be inhibited. Also, by inhibiting the binding between CTLA-4 and CD80 or CD86, the CTLA-4 axis immune checkpoint can be inhibited. Also, by inhibiting the binding between TIM-3 and galectin-9, the TIM-3 axis immune checkpoint can be inhibited. Also, by inhibiting the binding between LAG-3 and MHC class II molecules, the LAG-3 axis immune checkpoint can be inhibited. Also, by inhibiting the binding between VISTA and VSIG-3/IGSF11, the VISTA axis immune checkpoint can be inhibited. Also, by inhibiting the binding between SIRPα and CD47, the SIRPα axis immune checkpoint can be inhibited. In this manner, one or more immune checkpoints selected from the group consisting of the PD-1 axis immune checkpoint, the CTLA-4 axis immune checkpoint, the TIM-3 axis immune checkpoint, the LAG-3 axis immune checkpoint, the VISTA axis immune checkpoint, and the SIRPα axis immune checkpoint can be inhibited. Antibodies that inhibit the binding between two proteins can bind to the receptor or ligand. For example, antibodies that inhibit the PD-1 axis immune checkpoint include antibodies selected from the group consisting of anti-PD-1 antibody, anti-PD-L1 antibody, and anti-PD-L2 antibody (e.g., nivolumab, pembrolizumab, avelumab, atezolizumab, and durvalumab). Also, antibodies that inhibit the CTLA-4 axis immune checkpoint include antibodies selected from the group consisting of anti-CTLA-4 antibody, anti-CD80 antibody, and anti-CD86 antibody (e.g., ipilimumab and tremelimumab). Also, antibodies that inhibit the TIM-3 axis immune checkpoint include antibodies selected from the group consisting of anti-TIM-3 antibody and anti-galectin-9 antibody (e.g., MGB453). Also, antibodies that inhibit the VISTA axis immune checkpoint include antibodies selected from the group consisting of anti-VISTA antibody and anti-VSIG-3/IGSF11 antibody (e.g., JNJ-61610588). Also, antibodies that inhibit the SIRPα axis immune checkpoint include antibodies selected from the group consisting of anti-SIRPα antibody and anti-CD47 antibody (e.g., magrolimab, CC-90002). In addition, other molecules that inhibit the SIRPα axis immune checkpoint have been developed, including proteins that inhibit the binding between SIRPα and CD47 by combining the CD47-binding domain of human SIRPα with the Fc region of human IgG1 (e.g., Ontorpacept, Maprilpacept, and Evorpacept).

The subject preferably has cancer cells that express immune checkpoint molecules. Even if the tumor of the subject is in an immunosuppressive state (i.e., a non-inflammatory tumor (cold tumor)), the tumor can be converted into an inflammatory tumor (hot tumor), and therefore, any subject including those with non-inflammatory tumors or immune checkpoint inhibitor-resistant tumors may be eligible for treatment with immune checkpoint inhibitors. Accordingly, in one embodiment, the subject is a subject having resistance to immune checkpoint inhibitors. A subject having resistance to immune checkpoint inhibitors can be appropriately identified by a medical professional such as a physician based on the therapeutic course after administration. Alternatively, even when the subject does not express or barely expresses immune checkpoint molecules (i.e., when immune checkpoint inhibitors are ineffective), administration of the cell of the present disclosure may still be effective and may allow the immune system to be recruited to the tumor. Also, the interaction between administration of the cell of the present disclosure and immune cells may induce expression of immune checkpoint molecules in cancer, and combined administration of the cell of the present disclosure and immune checkpoint inhibitors may be effective. Cancer has a mechanism to express immune checkpoint molecules in order to suppress immune cells that come into contact with it. In one embodiment, a method for treating cancer in a subject having cancer cells that do not express or barely express immune checkpoint molecules comprises administering to the subject an effective amount of the cell of the present disclosure and administering an effective amount of an immune checkpoint inhibitor. The order of administration of the cell and the immune checkpoint inhibitor is not limited, but preferably, the cell is administered first and then the immune checkpoint inhibitor is administered. Administration of the cell of the present disclosure is considered to activate immunity of the cancer cells in the subject and/or enhance expression of immune checkpoint molecules in the cancer cells, thereby improving the efficacy of the immune checkpoint inhibitor. In one embodiment, the subject may be administered an immune checkpoint inhibitor when cells are administered to the subject and upregulation of immune checkpoint molecule expression in cancer cells is observed. The upregulation of immune checkpoint molecule expression in cancer cells can be confirmed by conventional methods such as immunohistochemical staining, flow cytometry, and enzyme-linked immunosorbent assay (ELISA). If, despite the administration of the cells of the present disclosure, the subject does not express or only minimally expresses immune checkpoint molecules (i.e., when immune checkpoint inhibitors are not effective), the immune checkpoint inhibitor may not be administered to the subject.

According to the present disclosure, the cell of the present disclosure for use in the above method is provided. According to the present disclosure, the composition of the present disclosure for use in the above method is provided.

According to the present disclosure, the use of the cell of the present disclosure in the manufacture of a medicament for use in the above method is provided. According to the present disclosure, the use of the cell of the present disclosure in the manufacture of a medicament for treating cancer is provided.

EXAMPLES

2. Materials and Methods

2.1. Materials

Dulbecco's Modified Eagle's Medium (DMEM), Roswell Park Memorial Institute-1640 (RPMI-1640) medium, antibiotic-antimycotic mixed stock solution, 4% paraformaldehyde phosphate-buffered saline (4%-FA PBS), and Dulbecco's phosphate-buffered saline (DPBS) were purchased from Nacalai Tesque Inc. (Kyoto, Japan). Fetal bovine serum (FBS) was purchased from Gibco Inc. (Waltham, MA, USA). Lipopolysaccharide (LPS) derived from Escherichia coli serotype O55:B5 and TritonX-100 were purchased from Sigma Aldrich Co. (St. Louis, MO, USA). Mouse IL-4, IL-13, TNF-$\alpha$, and IFN-$\gamma$ were purchased from Peprotech (Rocky Hill, NJ, USA). Cell Counting Kit-8 (CCK-8) was purchased from Dojindo Laboratories (Kumamoto, Japan). Lipofectamine 3000 and SuperScript III First-Strand Synthesis System were purchased from Thermo Fisher Scientific Inc. (Waltham, MA, USA). NucleoBond Xtra Maxi Plus EF, In-Fusion Snap Assembly Master Mix, PrimeSTAR Max DNA Polymerase, DNase I, and RNAiso plus were purchased from Takara Bio Inc. (Shiga, Japan). G418 sulfate was purchased from FUJIFILM Wako Pure Chemical Corporation (Osaka, Japan). Light-Cycler FastStart DNA Master SYBR Green Kit and Liberase DH Research Grade were purchased from Roche Diagnostics (Basel, Switzerland). Vivo-Track 680 (VT-680) was purchased from PerkinElmer, Inc. Phycoerythrin (PE)-labeled anti-mouse CD206 antibody, Brilliant Violet (BV) 421-labeled anti-mouse F4/80 antibody, Alexa Fluor 700-labeled anti-mouse CD86 antibody, Alexa Fluor 488-labeled anti-mouse CD11b antibody, FITC (fluorescein isothiocyanate)-labeled anti-mouse NKp46 antibody, PE-labeled anti-mouse NK1.1 antibody, Alexa Fluor 700-labeled anti-mouse CD3 antibody, Alexa Fluor 488-labeled anti-mouse CD8 antibody, Enzyme Linked Immuno Sorbent Assay (ELISA) MAX™ Deluxe Set Mouse TNF-$\alpha$, and Stop Solution for TMB Substrate were purchased from BioLegend, Inc. (San Diego, CA, USA). VersaLyse was purchased from Beckman Coulter Inc. (Brea, CA, USA). Aspartate aminotransferase (AST) Colorimetric Activity Assay Kit was purchased from Cayman Chemical (Ann Arbor, MI, USA). Goldenrod animal lancets were purchased from MEDIpoint, Inc. (Mineola, NY, USA). CD206/MRC1 (E6T5J) XP™ Rabbit mAb, CD86 (E5W6H) Rabbit mAb, and Ki-67 (D3B5) Rabbit mAb were purchased from Cell Signaling Technology, Inc. (Danvers, MA, USA). 3,3-Diaminobenzidine (DAB) and HRP-labeled rabbit antibody were purchased from Dako, Inc. (Glostrup, Denmark). pGL3-mArg1 promoter/enhancer (-31/-3810) was a gift from Peter Murray (Addgene plasmid #34571; http://n2t.net/addgene:34571; RRID: Addgene_34571) [36]. Other chemicals used were commercially available highest grade reagents.

2.2. Cell Culture

RAW264.7 mouse macrophages and CT-26 mouse colon carcinoma cells were purchased from American Type Culture Collection (ATCC). RAW264.7 cells were cultured in DMEM. CT-26 cells were cultured in RPMI-1640. All media were supplemented with 10% heat-inactivated FBS and 1% antibiotic-antimycotic mixed stock solution. Cells were cultured at 37°C in a humidified atmosphere of 5% $CO_2$ and 95% air. The medium was changed every two days, and when the cells reached 90% confluency, they were detached using a cell scraper and subcultured.

THP-1 human monocytic cells were purchased from JCRB. THP-1 cells were cultured in RPMI-1640. The medium was supplemented with 10% heat-inactivated FBS and 1% antibiotic-antimycotic mixed stock solution. Cells were cultured at 37°C in a humidified atmosphere of 5% $CO_2$ and 95% air. The medium was changed every two days, and when the cells reached 90% confluency, they were collected and subcultured.

2.3. Evaluation of Macrophages as Drug Carriers

2.3.1 In vitro Expression Assay

Expression of Arg1 mRNA in RAW264.7 cells was evaluated by quantitative real-time polymerase chain reaction (qPCR). RAW264.7 cells were plated in 6-well plates at a density of $1\times10^6$ cells/well in 2.5 mL of medium. After 24 hours, the medium was replaced with tumor-conditioned medium (TCM) prepared by culturing CT-26 cells ($3\times10^6$ cells, 2.5 mL medium) in 6-well plates for 24 hours. In other wells, 25 ng/mL IL-4 or IL-13, 200 ng/mL LPS, and 100 ng/mL TNF-$\alpha$ were supplemented. After culturing for 3, 6, 12, or 24 hours, cells were collected, and total RNA was extracted using RNAiso Plus according to the manufacturer's protocol. Subsequently, 5 $\mu$g of RNA was reverse transcribed using the SuperScript III First-Strand Synthesis System. PCR amplification was performed using the LightCycler FastStart DNA Master SYBR Green Kit. The following PCR conditions were used. Glyceraldehyde-3-phosphate dehydrogenase (GAPDH) was used as a housekeeping gene, and the relative expression of the target gene was determined using the comparative CT method [37]. Primers for Arg1 and GAPDH were purchased from Fasmac Co., Ltd. All primer sequences are shown in Table 1 [33,38].

[Table 1]

Table 1: Primer sequences for PCR

| Name | Sequence (5'→3') |
| --- | --- |
| GAPDH F (SEQ ID NO: 1) | CCCAGCAAGGACACTGAGCAAG |
| GAPDH R (SEQ ID NO: 2) | GGTCTGGGATGGAAATTGTGAGGG |
| Arg1 F (SEQ ID NO: 3) | CAGAAGAATGGAAGAGTCAG |
| Arg1 R (SEQ ID NO: 4) | CAGATATGCAGGGAGTCACC |

Single cell RNA sequence data of human THP-1-derived macrophages were selected and retrieved from the NCBI database GEO Data sets (https://www.ncbi.nlm.nih.gov/). After conversion to text format, heatmap analysis was performed using iDEP.96 (http://bioinformatics.sdstate.edu/idep96/).

Gene expression analysis of human macrophages was conducted as follows. THP-1 cells were differentiated into macrophages by adding 20 ng/mL of phorbol 12-myristate 13-acetate (PMA) to the culture medium. THP-1 cells were cultured at a density of $5\times10^6$ cells/well in 2.5 mL of medium in a 6-well plate. After 16 hours, macrophages were stimulated with IL-4 and IL-13 (25 ng/mL each) to polarize them into M2 macrophages. For polarization into M1 macrophages, the cells were stimulated with LPS (200 ng/mL) and IFN-$\gamma$ (100 ng/mL) for 4 hours. For CCL-22, LOX, CISH, FCER15, ALOX15, F13A1, IDO1, MMP1, ALDH1A2, CD209, TGM2, VCAM1, MMP12, and SPINT2, cells were collected after 72 hours of culture; for CCL-22, CISH, ALOX15, and TGM2, cells were collected after 24, 28, and 72 hours of culture. Total RNA was extracted using RNAiso Plus according to the manufacturer's protocol. Subsequently, 5 $\mu$g of RNA was reverse transcribed using the SuperScript III First-Strand Synthesis System. PCR amplification was performed using the LightCycler FastStart DNA Master SYBR Green Kit. The following PCR conditions were used. GAPDH (glyceraldehyde-3-phosphate dehydrogenase) was used as a housekeeping gene, and the relative expression levels of target genes were determined by the comparative $C_T$ method. The primers used are as follows.

[Table 2]

| Table 2: Primer sequences that were used | |
| --- | --- |
| Name | Sequence (5'→3') |
| GAPDH F (SEQ ID NO: 5) | GTCTCCTCTGACTTCAACAGCG |
| GAPDH R (SEQ ID NO: 6) | ACCACCCTGTTGCTGTAGCCAA |
| CCL-22 F (SEQ ID NO: 7) | GAGCATGGATCGCCTACAG |
| CCL-22 R (SEQ ID NO: 8) | CAGACGGTAACGGACGTAATC |
| CISH F (SEQ ID NO: 9) | GCATAGCCAAGACCTTCTCCTAC |
| CISH R (SEQ ID NO: 10) | ACGTGCCTTCTGGCATCTTCTG |
| ALOX15 F (SEQ ID NO: 11) | ACCTTCCTGCTCGCCTAGTGTT |
| ALOX15 R (SEQ ID NO: 12) | GGCTACAGAGAATGACGTTGGC |
| TGM2 F (SEQ ID NO: 13) | TGTGGCACCAAGTACCTGCTCA |
| TGM2 R (SEQ ID NO: 14) | GCACCTTGATGAGGTTGGACTC |
| LOX F (SEQ ID NO: 15) | GATACGGCACTGGCTACTTCCA |
| LOX R (SEQ ID NO: 16) | GCCAGACAGTTTTCCTCCGCC |
| FCER1A F (SEQ ID NO: 17) | GTGGAGAATACAAATGTCAGCACC |

(continued)

| Table 2: Primer sequences that were used | |
|---|---|
| Name | Sequence (5'→3') |
| FCER1A R (SEQ ID NO: 18) | CTCCATCACCACCTCAGCAGAG |
| F13A1 F (SEQ ID NO: 19) | CAACAGCCACAACCGTTACACC |
| F13A1 R (SEQ ID NO: 20) | CTTGGATCAGCACCGCCTCTTT |
| ALDH1A2 F (SEQ ID NO: 21) | GAGTAACTCTGGAACTTGGAGGC |
| ALDH1A2 R (SEQ ID NO: 22) | ATGGACTCCTCCACGAAGATGC |
| CD209 F (SEQ ID NO: 23) | GCAGTCTTCCAGAAGTAACCGC |
| CD209 R (SEQ ID NO: 24) | GCTCTCCTCTGTTCCAATACTGC |
| VCAM1 F (SEQ ID NO: 25) | GATTCTGTGCCCACAGTAAGGC |
| VCAM1 R (SEQ ID NO: 26) | TGGTCACAGAGCCACCTTCTTG |
| MMP12 F (SEQ ID NO: 27) | GATGCTGTCACTACCGTGGGAA |
| MMP12 R (SEQ ID NO: 28) | CAATGCCAGATGGCAAGGTTGG |
| SPINT2 F (SEQ ID NO: 29) | GTGCCTCAAGAAATGTGCCACTG |
| SPINT2 R (SEQ ID NO: 30) | GGAGTGGTCTTCAGAATCCTGC |

2.3.2 In vivo evaluation of macrophage migration and hepatotoxicity

All animal experiments were conducted with the approval of the Institutional Animal Care and Use Committee of Kyushu University. Female BALB/cAJcl mice aged 6-8 weeks were obtained from Kyudo Co., Ltd. (Saga, Japan). After one week of acclimatization, CT-26 cells ($1 \times 10^6$ cells in 100 $\mu$L PBS) were subcutaneously (s.c.) injected into the abdomen. On day 5 after tumor implantation, VT-680-labeled RAW264.7 macrophages ($1 \times 10^6$ cells in 100 $\mu$L PBS) were administered intravenously (i.v.). In vivo fluorescence imaging was performed on days 1, 4, and 7 after macrophage administration using the IVIS Spectrum (IVIS Lumina II, Xenogen Corp.) to visualize migration of macrophages to the tumor tissue. Subsequently, on days 4 and 7 after macrophage administration, blood was collected using animal lancets. After euthanizing the mice, the tumor, liver, spleen, kidney, and lung were harvested, and accumulation of macrophages was evaluated using the IVIS Spectrum. Plasma AST activity was analyzed using an AST assay kit according to the manufacturer's protocol.

2.3.3 Evaluation of macrophage polarization by flow cytometry

Tumor and liver were collected and washed with PBS. After 60 minutes, the solution was filtered through a 100 $\mu$m filter and centrifuged at 300 $\times$ g for 5 minutes. After removal of the supernatant, 4 mL of VersaLyse was added and incubated at room temperature for 5 minutes. After incubation, the sample was centrifuged at 300 $\times$ g for 5 minutes and resuspended in FACS buffer (DPBS containing 2% FBS). The liver was homogenized on a 100 $\mu$m filter using a plunger and suspended in cold DPBS. After centrifugation at 200 $\times$ g for 5 minutes, 4 mL of VersaLyse was added and incubated at room temperature for 5 minutes. After incubation, the sample was centrifuged at 200 $\times$ g for 5 minutes and resuspended in FACS buffer. After resuspension, fluorescent-labeled antibodies (Alexa Fluor 488-conjugated anti-mouse CD11b antibody, BV421-conjugated anti-mouse F4/80 antibody, and PE-conjugated anti-mouse CD206 antibody) were added and incubated at 4°C for 60 minutes. After incubation, the cells were washed and resuspended in FACS buffer. Expression of CD206 (an M2 macrophage marker) in the administered macrophages (CD11b$^+$, F4/80$^+$, VT-680$^+$) was evaluated by flow cytometry (CytoFLEX, Beckman Coulter).

2.4. Production and in vitro characterization of MacTrigger

2.4.1 Preparation of plasmid and transfection into macrophages

As an M2 macrophage-specific promoter, the mArg1 promoter/enhancer (-31/-3810) from pGL3-mArg1 was amplified by PCR using PrimeSTAR Max DNA Polymerase and inserted downstream of the CMV promoter-target gene region of pcDNA3.1 using In-Fusion Snap Assembly Master Mix. A gene encoding mouse TNF-$\alpha$, as an inflammatory

cytokine, was purchased from Integrated DNA Technology, Inc. (Coralville, IA, USA) and cloned downstream of the M2 macrophage-specific Arg1 promoter to generate pcDNA3.1-mArg1p-TNF-$\alpha$. In addition, a mouse TNF-$\alpha$ gene and enhanced green fluorescent protein (EGFP) coding sequence were inserted downstream of the CMV promoter to generate pcDNA3.1-CMVp-TNF-$\alpha$ and pcDNA3.1-CMVp-EGFP, respectively. Plasmid DNA was extracted using NucleoBond Xtra Maxi Plus EF. Transfected RAW264.7 cells expressed TNF-$\alpha$ in response to the Arg1 promoter (Arg1p-TNF$\alpha$), or in response to the CMV promoter (CMVp-TNF$\alpha$), or expressed EGFP in response to the CMV promoter (CMVp-EGFP). RAW264.7 cells were seeded at a density of $2.0 \times 10^5$ cells/well in a 24-well plate. After 24 hours, Arg1p-TNF-$\alpha$, CMVp-TNF-$\alpha$, or CMVp-EGFP vectors were transfected into RAW264.7 cells using Lipofectamine 3000 reagent according to the manufacturer's protocol. Six hours after transfection, the medium was replaced with fresh medium. The cells were then treated with 400 $\mu$g/mL G418 to obtain stable cell lines. In the same manner (except using other genes in place of TNF-$\alpha$), stable cell lines expressing IL-1$\beta$ (NCBI Reference Sequence: NP_032387.1), IL-2 (NCBI Reference Sequence: NP_032392.1), IL-6 (GenBank: ABG81953.1), IL-12 (IL-12$\alpha$ subunit: NCBI Reference Sequence: NP_001152896.2, IL-12$\beta$ subunit: NCBI Reference Sequence: NP_001290173.1), and IFN-$\gamma$ (NCBI Reference Sequence: NP_032363.1) were also generated. Non-transfected macrophages, CMVp-TNF-$\alpha$-transfected macrophages, CMVp-EGFP-transfected macrophages, and Arg1p-TNF-a-transfected macrophages were named Mac(UTD), Mac(CMVp), Mac(EGFP), and MacTrigger, respectively.

A human-type MacTrigger was also constructed. Specifically, the procedure was as follows. The region of pcDNA3.1 excluding the promoter-MCS (multi cloning site) was amplified by PCR using PrimeSTAR Max DNA Polymerase to prepare a linearized vector. After the amplification of the vector, the product was treated with DpnI restriction enzyme. A synthetic gene encoding human TNF-$\alpha$ (Sequence ID No. 32) operably linked to the CCL-22 promoter (Sequence ID No. 31) (CCL22p-hTNF-$\alpha$) was purchased from IDT. The purchased synthetic DNA was amplified by PCR in the same manner as described above. The amplified linearized vector and synthetic DNA were mixed with In-Fusion Snap Assembly Master Mix, incubated at 60°C for 15 minutes, and plasmid DNA was constructed by the In-Fusion method. The resulting plasmid DNA was introduced into JM109 competent cells and cultured overnight on LB agar medium containing ampicillin. A single colony was picked and cultured in 5 mL of LB medium for 6 hours. Plasmid DNA was extracted from the cultured E. coli using QIAGEN plasmid extraction reagents. After confirming that the desired sequence was obtained by sequence analysis, the cells were re-transformed into JM109 and cultured. After culture, plasmid DNA was extracted under endotoxin-free conditions using the NucleoBond™ Xtra Maxi Plus EF Kit.

# EP 4 663 750 A1

[Table 3]

| Table 3: Primer sequences that were used |
|---|
| Human CCL-21 promoter (SEQ ID NO: 31) |
| GGCGCATGCCATCACACCCGGCCAATTTTTGTATTTTTGGTAGAGATGGGGTTTCTCCATGTTGGCCAGGCTGGTCTT GAACTCCTGACCTCAGGTGATCCACCTGCCTGGGCCTTCCAAAGTGCTGGGATTACAGTCATGAATCACTGCACCCAG CCCATGTGTTCTGAGCACCCAACATGTGCCTGATCCTCAGAGGGCCCTGGGAATGCCAAGAAAAATGAAGGGAGACCC CAGCCTCCTAGAGCTTGGAGTCTGACTTCTAGGGAGGGGCTTGAGAAAGAGAAAACCTGACCCAGTCTGCTCAGGGCC ACATTCCAGGTCCCCTAGAAGGCTTTGAAGTCCTGAGAGGGTGTGCTTACCAAGGACATGACATTTCAAACAGCTTTG ACGCATAAGTAGGAGTTTATGTGGTAGAGATGTGGAAAGTGACATTAAAGGCCAGGGACAGGCCGCAGTCCCTTTTGA GGAAGTGTGAGGAGTGTGGTGTGATCCCTCAGGGGAAGGAGACCAGTGGGGAAAGGCCTGTCCGGAAACGGGGCCTTG AAGGCCACAGACAGGAGCCCGGGACCTGCCTTTAGGTGAATGGGGAGCCACGGCAGGGTTCTGAAGCAGGAGAGAGAG TGTGTAGCTCTGGACCACTCACGCTGGCTGCTGCAGGGTGGGGATGGCATTGAGGAGGCCAGCATCCCAGCCTGAGGT GGAGGTGGCCTGGACTGGGCAGGAACAGTGGGGTTGGAGAGAAGGAGCAGATCTGAGAGAGATTTAGGAAGCAGAATC AACGGGACATGGACATGGTGAGGGACTGAGACAGGAGTGGGGGAGGGAGAGCCCGGCAGCCACTGGGAAGGAAGATCA GGCAGGACAGAAGTGTGGGTAGATGCTTTCCATAAACCGTTGAGAAATCCACGACCTGGCCCAAATGTGGACTTCACG AGAAGCCCCAGATGGGTCAATGGGTCCATAGGCCTCTCCCATTGAAGAGAGACCAGTAATGCCGTCATTAAGGGTACA AGACAGGCCTCAATTCTAGGTGAGGGTTGGTCCACGTTCTCACTTGATTTACAACCAGTGAAGTCAACAGAGCTGTGA AACCATCTCCCCCATTTCCAGATGGGGAAACCAAGTCCCAGAGACACCCAGACATGGCCAAGGCCACACAGTGCAGAG ACCCTGGGGAGCACAAGCCTCTAAGAGGGGGATTTTACAAGTCTGTCTGAAACCAGAGGCGAGGACAGAGTTGAGGGG GGGTCCCCTCTGAGTGGTTCCCCGCCAAAGAGAATTTCTCCACTTTGGAGGAAGTGGGAGGTAGTTCTTCTTTTGAGA TGTGAATGTCAAGTGACTCTGGGCACCCCGGTGACTAAGAGGTAAGTAAGTGAGGCTTGTGGGTGGAGCCAGCACCTT AAATAGCAGGTCTTCCTATGTCCCTTTGCAGACACCTGGGCT |
| Human TNF-α (SEQ ID NO: 32) |
| ATGTACAGGATGCAACTCCTGTCTTGCATTGCACTAAGTCTTGCACTTGTCACAAACAGTTCAACTGAATCCATGATA AGAGACGTAGAACTTGCCGAAGAAGCCCTCCCCAAAAAGACAGGTGGCCCCCAAGGAAGTCGGAGGTGCCTGTTCCTG TCACTTTTTTCATTTCTTATAGTGGCCGGTGCTACAACCTTGTTCTGTCTCCTGCACTTTGGTGTGATCGGTCCCCAA CGAGAAGAGTTTCCTCGAGACCTTTCCCTGATAAGCCCCTTGGCCCAGGCCGTGAGGTCATCTAGTAGGACACCAAGC GATAAGCCTGTAGCACATGTCGTTGCCAACCCCCAAGCAGAAGGACAGCTCCAGTGGCTGAACCGGCGAGCTAATGCA TTGTTGGCTAACGGAGTAGAACTTCGAGATAACCAACTCGTAGTTCCATCCGAAGGTTTGTACTTGATTTATTCCCAA GTTCTTTTTAAGGGGCAAGGTTGTCCCTCAACCCACGTATTGTTGACTCATACAATATCACGAATAGCTGTTAGCTAT CAAACAAAAGTAAATTTGCTTTCCGCTATCAAGTCTCCTTGTCAACGGGAAACTCCAGAGGGCGCCGAAGCTAAACCT TGGTACGAGCCCATTTACCTCGGGGGCGTCTTCCAACTGGAAAAGGGGGACCGCTTGTCTGCCGAGATCAACCGGCCC GATTATTTGGATTTTGCCGAGAGTGGGCAGGTGTATTTTGGCATCATTGCTCTCTAA |

To transfect the constructed plasmid into THP-1 cells, THP-1 cells were seeded at a density of $5.0 \times 10^6$ cells/well in a 24-well plate. After 24 hours, the CCL-22-TNF-α vector was transfected into THP-1 cells using TransIT-Jurkat reagent according to the manufacturer's protocol. Six hours after transfection, the medium was replaced with fresh medium. The cells were treated with G418 to obtain a stable cell line. By stimulating this cell line with 20 ng/mL PMA for 16 hours, human-type MacTrigger was obtained.

2.4.2 In vitro evaluation of TNF-α production

MacTrigger cells were seeded in a 24-well plate at a density of $1 \times 10^5$ cells/well in 500 μL of medium. After 24 hours, the medium was replaced with fresh medium, TCM, or medium containing 25 ng/mL IL-4 or IL-13. At 24, 48, and 72 hours, the supernatant was collected and TNF-α concentration was measured using ELISA MAX™ Deluxe Set Mouse TNF-α according to the manufacturer's protocol. TNF-α production by human-type MacTrigger cells was evaluated in the same manner.

2.4.3 In vitro evaluation of the inflammation-inducing ability of MacTrigger

Mac(EGFP) cells were seeded in a 24-well plate at a density of $2\times10^5$ cells/well in 500 µL of medium. After 24 hours, 25 ng/mL IL-13 was added to the medium. After another 24 hours, the cells were collected and seeded again in 24-well plates at a density of $1\times10^5$ cells/well in 500 µL of medium containing IL-13. Next, Mac(UTD), Mac(CMVp), or MacTrigger cells were individually seeded in 24-well plates containing DMEM with IL-13 at densities of $1\times10^5$ cells/well, $2\times10^5$ cells/well, or $4\times10^5$ cells/well (corresponding to effector/target ratios of 1, 2, or 4, respectively). After culturing for 72 hours, the cells were collected and incubated with PE-conjugated anti-mouse CD206 antibody and Alexa Fluor 700-conjugated anti-mouse CD86 antibody. After 60 minutes of incubation at 4°C, the proportions of CD206+ cells / EGFP+ cells and CD86+ cells / EGFP+ cells were analyzed using a CytoFLEX.

2.4.4 In vitro evaluation of MacTrigger cell survival time

MacTrigger cells were seeded in 96-well plates at a density of $5\times10^3$ cells/well and 200 µL of medium was added. After 24 hours, the medium was replaced with fresh medium, TCM, or medium containing 25 ng/mL IL-4 or IL-13 and cultured for 1 to 6 days. After culturing, cell viability was evaluated using CCK-8 according to the manufacturer's protocol. Absorbance at 450 nm was measured using the Infinite™ 200 PRO M Plex (manufactured by TECAN). Cell viability was calculated using the following formula:

[Formula 1]

$$\% \text{ cell viability} = \frac{A_1 - A_0}{A_{ctrl} - A_0} \times 100$$

Here, A1 is the absorbance in the presence of cytokines or TCM, Actrl is the absorbance in the absence of cytokines or TCM, and A0 is the absorbance in the absence of cytokines and cells.

2.5. Antitumor effects of artificial macrophages in tumor-bearing mice

The antitumor effects of artificial macrophages were evaluated in tumor-bearing mice. CT-26 cells, 4T1 cells, or Colon-26 cells ($1.0\times10^6$ cells in 100 µL PBS each) were subcutaneously injected into the abdominal region of 8-week-old male BALB/c mice. On day 5 (Day 0) or day 10 after tumor implantation, PBS (100 µL), Mac(UTD), Mac(CMVp), or MacTrigger ($1.0\times10^6$ cells in 100 µL PBS) were administered intravenously. Tumor volume and body weight were measured every two days, and blood was collected every four days.

[Formula 2]

$$\text{Tumor volume} = \frac{(Major\ axis)\times(Minor\ axis)^2}{2}$$

Tumor volume on day 0 of administration was set as 1, and the relative tumor volume of each group was calculated accordingly. The experiment was terminated when the tumor volume in the PBS-treated group reached 1000 mm³ (humane endpoint), and all mice were sacrificed. After sacrifice, tumor tissue and liver were excised, and liver weight was measured. Extraction of cells from tumor tissue was performed according to the protocol described in 2.3.3. After resuspension in FACS buffer, fluorescently labeled antibodies (Alexa Fluor 488-labeled anti-mouse CD11b antibody, BV421-labeled anti-mouse F4/80 antibody, PE-labeled anti-mouse CD206 antibody, Alexa Fluor 700-labeled anti-mouse CD86 antibody, or Alexa Fluor 700-labeled anti-mouse CD3 antibody and Alexa Fluor 488-labeled anti-mouse CD8 antibody, or FITC-labeled anti-mouse NKp46 antibody and PE-labeled anti-mouse NK1.1 antibody) were added and incubated at 4°C for 60 minutes. After incubation, cells were washed twice and resuspended in FACS buffer. Expression of CD206, CD86, CD3, CD8, NK1.1, and NKp46 was evaluated by flow cytometry.

2.6. Histological analysis

Hematoxylin-eosin (HE) staining and immunohistochemistry (IHC) were performed according to standard protocols. Tumor and liver tissues were fixed with 4%-PFA PBS. For IHC, paraffin-embedded tissue sections were used for staining of CD86, CD206, and Ki67. Tumor tissues were deparaffinized, hydrated, and heated in citrate buffer (pH 6.0) at 95°C for 15 minutes, followed by blocking with 3% skim milk for 10 minutes. The slides were incubated overnight with anti-CD86 antibody (1:100), anti-CD206 antibody (1:200), or anti-Ki67 antibody (1:200). After washing with PBS, the slides were incubated for 30 minutes in 0.3% hydrogen peroxide-methanol buffer to eliminate endogenous peroxidase activity. After washing with PBS, HRP-conjugated secondary antibodies were added and incubated for 30 minutes. After washing with PBS, DAB was added as a enzymatic substrate. For HE staining, tumor and liver tissues were deparaffinized and washed with water. Hematoxylin was added and incubated for 5 minutes. After incubation, the slides were washed and hydrated. Eosin was added and incubated for 5 minutes, followed by washing. Images of HE and IHC-stained sections were observed using an all-in-one fluorescence microscope (BZ-X800, KEYENCE, Osaka, Japan).

2.7. Statistical Analysis

Statistical significance was assessed using a two-tailed Student's t-test. Symbols *, **, and *** indicate p-values less than 0.05, 0.01, and 0.005, respectively, and N.S. indicates no significant difference.

Results

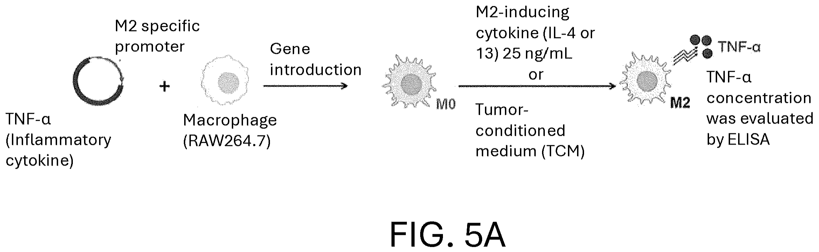

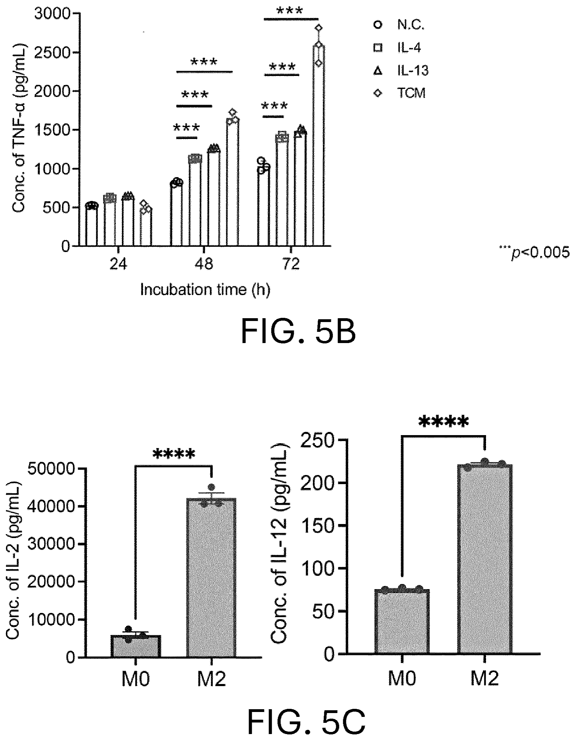

As shown in the scheme of Figure 5A, a gene encoding an inflammatory cytokine driven by an M2 macrophage-specific promoter was introduced into macrophages to generate M0 macrophages (MacTrigger). As examples of inflammatory cytokines, TNF-$\alpha$, IL-1$\beta$ (NCBI Reference Sequence: NP_032387.1), IL-2 (NCBI Reference Sequence: NP_032392.1), IL-6 (GenBank: ABG81953.1), IL-12 ($\alpha$-subunit: NCBI Reference Sequence: NP_001152896.2 and $\beta$-subunit: NCBI Reference Sequence: NP_001290173.1), and IFN-$\gamma$ (NCBI Reference Sequence: NP_032363.1) were used. As shown in Figure 5A, MacTrigger was cultured in the presence of IL-4 or IL-13 as M2 macrophage-inducing cytokines, or in the presence of tumor-conditioned medium (TCM) from cancer cell culture. At 24, 48, and 72 hours after the start of culture, culture supernatants were collected, and the amounts (concentrations) of TNF-$\alpha$, IL-2, and IL-12 produced by the cells were determined according to 2.4.2. The results are shown in Figure 5B. As shown in Figure 5B, in the presence of IL-4 or IL-13, which are M2 macrophage-inducing cytokines, MacTrigger produced TNF-$\alpha$. MacTrigger also produced TNF-$\alpha$ in the presence of TCM. These findings suggest that MacTrigger differentiated into M2 macrophages in the presence of IL-4 or IL-13 or TCM and thereby produced TNF-$\alpha$. Furthermore, the fact that M2 differentiation was promoted in the presence of TCM suggests that the tumor tissue provides an environment that promotes polarization into M2 macrophages. As shown in Figure 5C, MacTrigger incorporating IL-2 and IL-12 also produced these cytokines specifically in M2, indicating that these MacTriggers will also release cytokines specifically in tumor tissues. Thus, by driving cytokine production under an M2-specific promoter, MacTrigger accumulates in the tumor, polarizes into M2 macrophages in the tumor tissue, and begins to release cytokines. If the cytokine is an inflammatory cytokine, MacTrigger is expected to convert the tumor into an inflammatory environment.

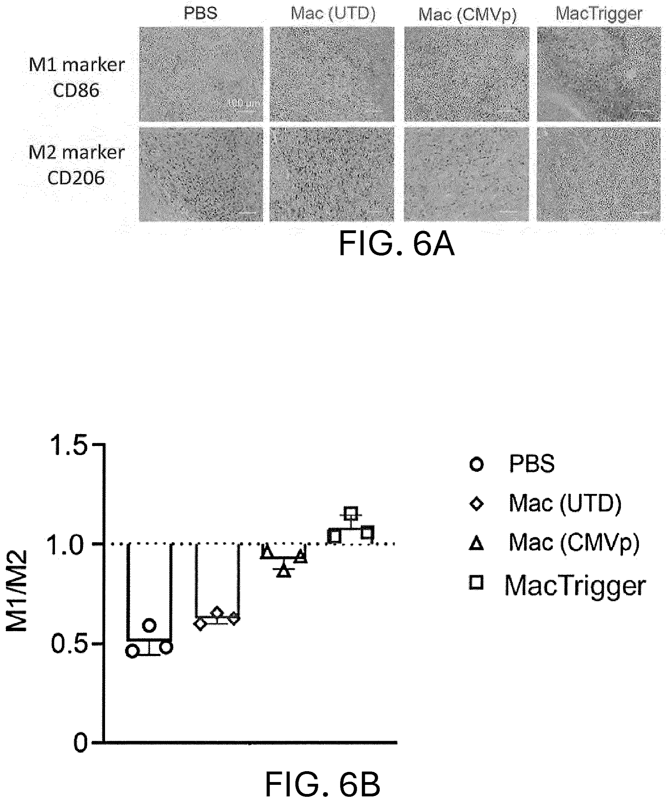

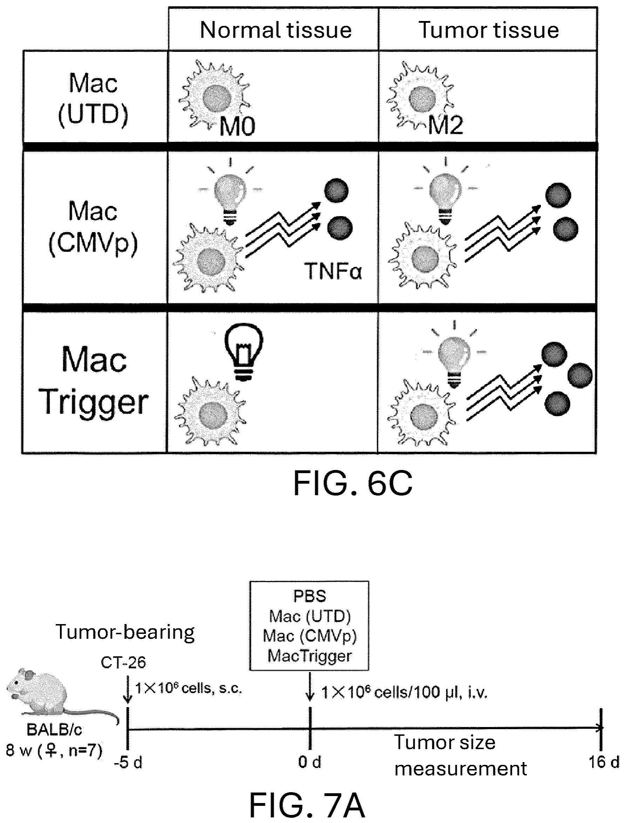

According to the administration method described in 2.4.4, Mac(UTD), Mac(CMVp), or MacTrigger was administered to tumor-bearing mice. On day 16 after administration, tumor tissue was collected, and expression of CD86 and CD206 was confirmed by immunohistochemistry. The results are shown in Figure 6A. As shown in Figure 6A, expression of CD86, an M1 marker, was markedly increased in the MacTrigger-administered group. Based on the above staining results, the ratio of M1 macrophages to M2 macrophages (M1/M2) was determined. As shown in Figure 6B, in the groups administered with Mac(UTD) or Mac(CMVp), the M1/M2 ratio was less than 1, indicating M2 predominance; however, in the MacTrigger group, the M1/M2 ratio exceeded 1, indicating M1 predominance. This suggests that only in the MacTrigger-administered group did the tumor tissue transition to an inflammatory state. As shown in Figure 6C, MacTrigger did not release inflammatory cytokines in normal tissue but differentiated into M2 macrophages only in tumor tissue, secreted inflammatory cytokines in the tumor tissue, converted the tumor into an inflammatory state, and induced an increase in inflammatory cells such as M1 macrophages within the tumor tissue.

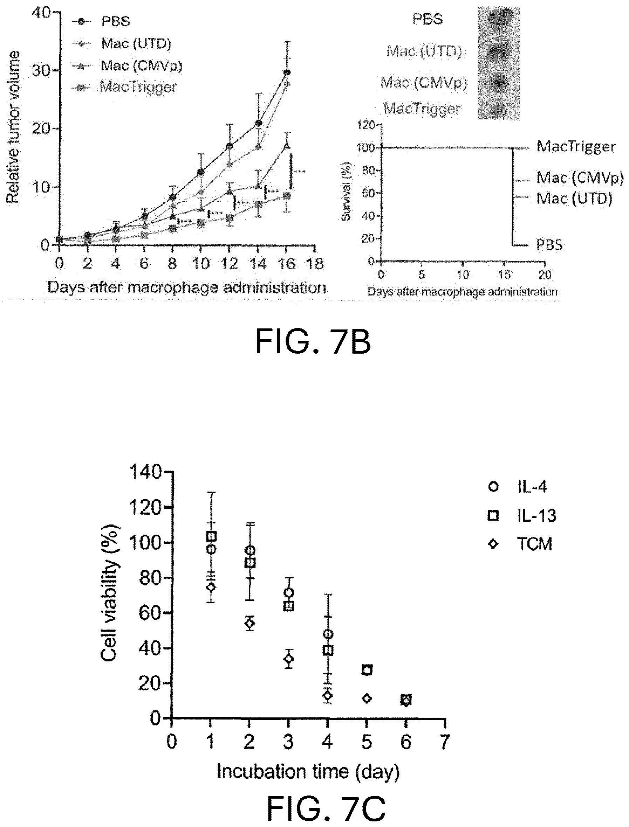

According to the scheme shown in Figure 7A and the method described in 2.4.4, Mac(UTD), Mac(CMVp), or MacTrigger was administered to tumor-bearing mice. Tumor size was measured up to 16 days after administration. As shown in the left panel of Figure 7B, the MacTrigger-administered group exhibited the greatest suppression of tumor size increase. Also, survival rates of the tumor-bearing mice were monitored, and as shown in the right panel of Figure 7B, most mice in the PBS group died, whereas no deaths were observed in the MacTrigger group. Thus, conversion of the tumor tissue to an inflammatory state by MacTrigger administration significantly suppressed both tumor growth and mortality.

In an in vitro experiment, MacTrigger was cultured in the presence of IL-4 or IL-13, or in the presence of TCM, and the viability of MacTrigger was evaluated using the WST-8 assay. The results are shown in Figure 7C. As shown in Figure 7C, MacTrigger was found to be nearly completely eliminated within approximately 4 days after administration. The death of MacTrigger is thought to be due to the effect of the TNF-$\alpha$ it secretes. Considering the lifespan of MacTrigger, the continued suppression of tumor size increase and the maintenance of survival ratio shown in Figure 7B persisted even after MacTrigger had died, suggesting that these effects are due to the persistence of the inflammatory environment in the tumor tissue induced by MacTrigger. Consistent with this, M1 macrophages were dominant in tumor tissue 16 days after MacTrigger administration (see Figures 6A and 6B).

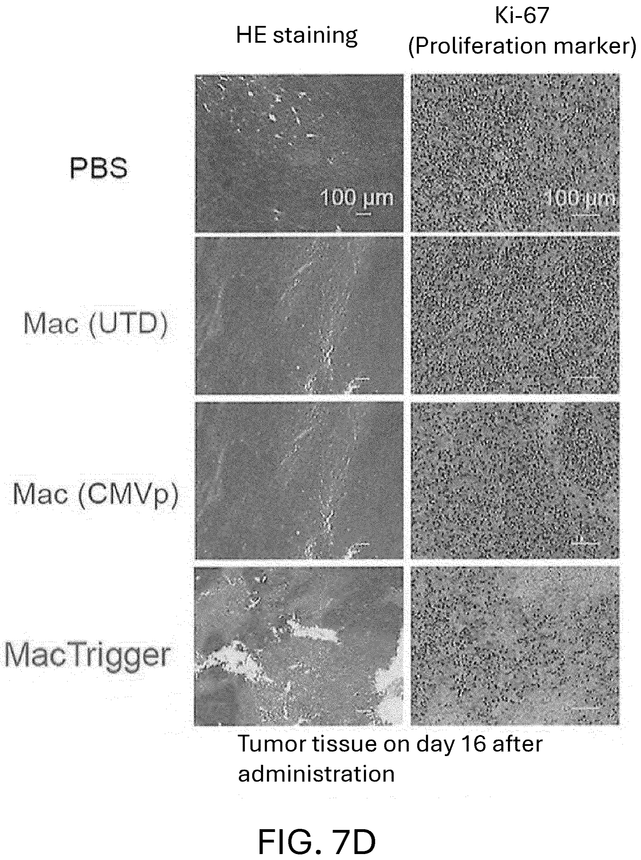

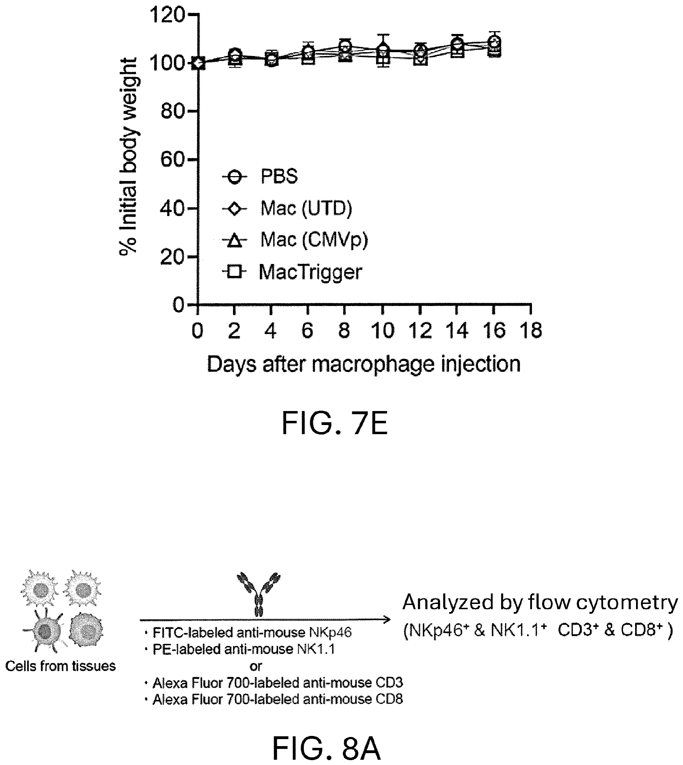

Tumor tissue was excised 16 days after MacTrigger administration, and expression of the proliferation marker Ki67 was confirmed by immunohistochemical staining. The results are shown in Figure 7D. As shown in Figure 7D, in the MacTrigger-administered group, expression of Ki67 was clearly reduced compared to the other groups, suggesting that cell proliferation in the tumor tissue was suppressed. The body weight of tumor-bearing mice was monitored over time. As shown in Figure 7E, no body weight loss due to MacTrigger administration was observed.

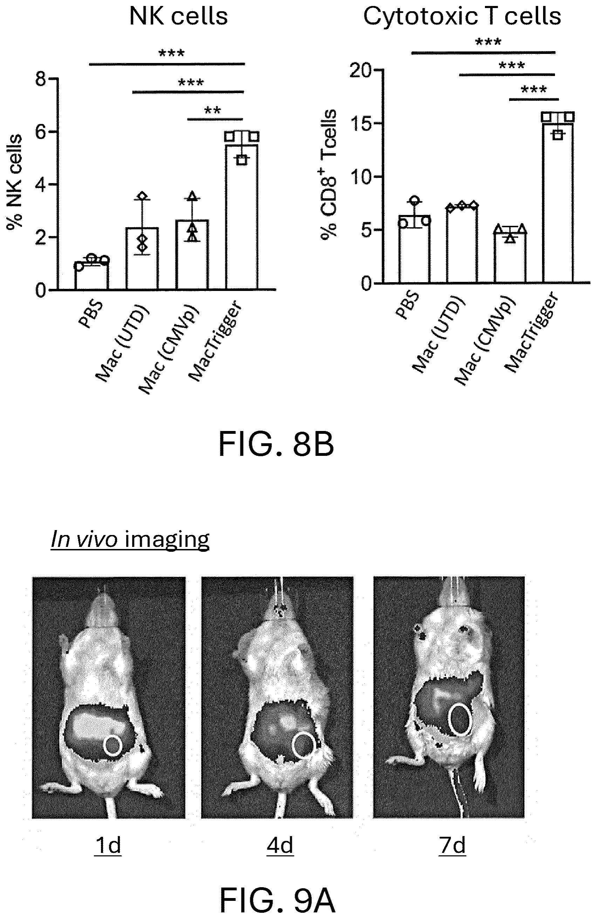

The number of immune cells in the tumor tissue of individuals administered with MacTrigger was evaluated. Specifically, tumor tissue was collected from mice 16 days after MacTrigger administration, and NK cells and cytotoxic T cells were gated by flow cytometry as described in Figure 8A. The results are shown in Figure 8B. As shown in Figure 8B, in the MacTrigger-administered group, the proportions of NK cells and cytotoxic T cells in tumor tissue were markedly increased. This demonstrates that the inflammation in tumor tissue induced by MacTrigger administration led not only to an increase in M1 macrophages in the tumor tissue, but also to an increase in NK cells and cytotoxic T cells.

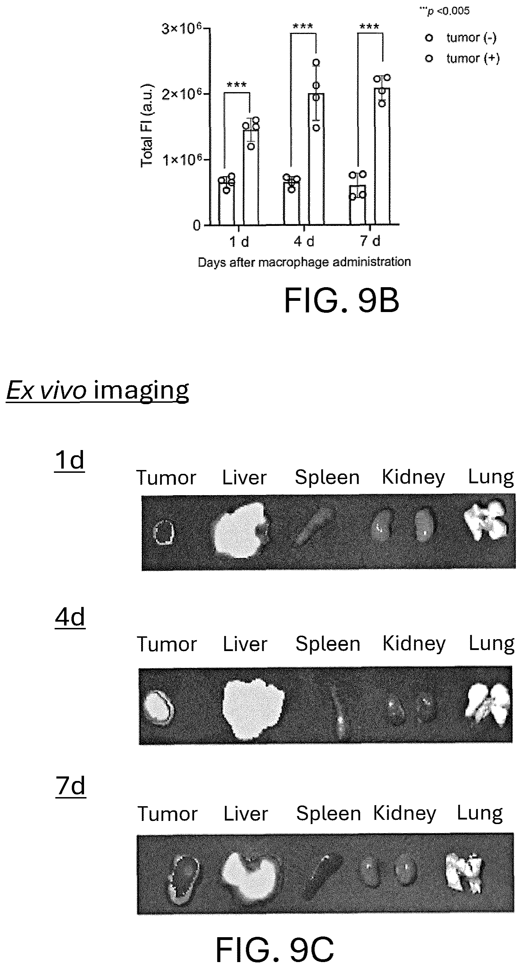

The biodistribution of macrophages in mice after administration of VT-680-labeled macrophages was examined. Specifically, as described in 2.3.2, VT-680-labeled macrophages were administered to tumor-bearing and non-tumor-bearing mice, and *in vivo* imaging was performed using the IVIS Spectrum on days 1, 4, and 7 after administration. The results, shown in Figures 9A and 9B, indicated that macrophages accumulated in the tumor tissue.

Tumor tissue and various organs were harvested from mice on days 4 and 7 after macrophage administration, and macrophage distribution was analyzed based on VT-680 signal. As shown in Figure 9C, macrophages accumulated predominantly in the tumor and liver. These findings suggest that macrophages migrated to the liver.

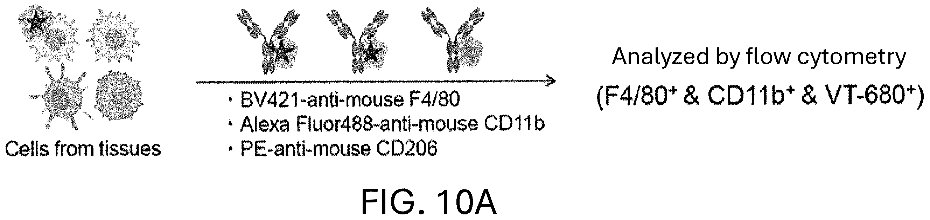

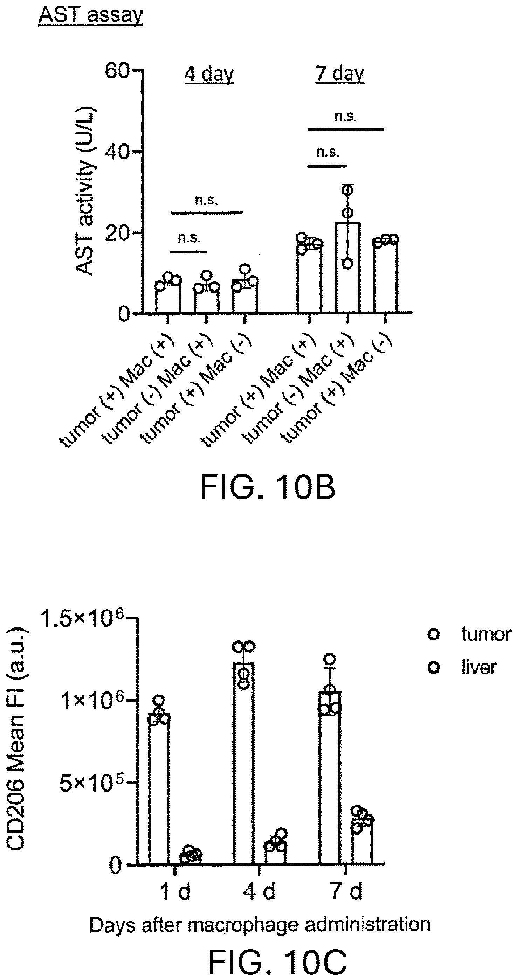

Hepatotoxicity due to macrophage accumulation in the liver was evaluated. As described in 2.3.2, macrophages were administered to tumor-bearing and non-tumor-bearing mice, and plasma AST activity was measured on days 4 and 7 after administration. As shown in Figure 10B, there was no significant difference in AST levels between the macrophage-administered group and the non-administered group. This suggests that although macrophages accumulate in the liver, they do not cause liver damage. The level of the M2 macrophage marker CD206 in tumor tissue and liver was examined. As shown in Figure 10C, a marked increase in M2 macrophages was observed in tumor tissue. In contrast, no marked increase in M2 macrophages was observed in the liver. This suggests that macrophages polarize into M2 macrophages only within tumor tissue.

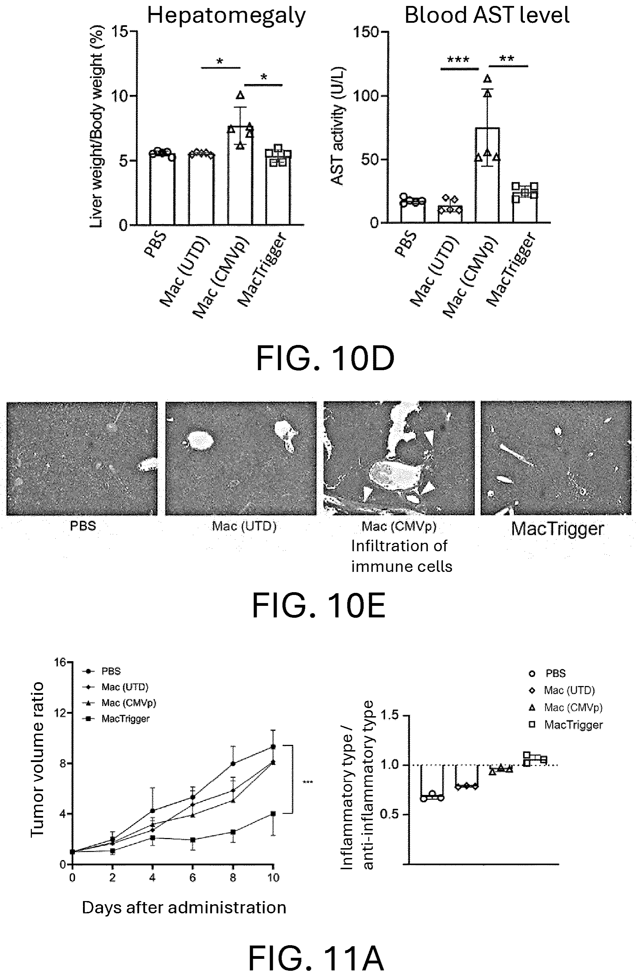

To verify the effect of MacTrigger administration on the liver, liver weight and AST levels of mice 16 days after administration were examined, and as shown in Figure 10D, no significant difference was observed between the MacTrigger-administered group and the PBS-administered group. Also, as shown in Figure 10E, no infiltration of immune cells was observed in liver tissue sections from the MacTrigger-administered group. These results clarified that MacTrigger does not significantly differentiate into M2 macrophages in the liver and does not induce hepatotoxicity. In contrast, in the Mac(CMVp)-administered group, the administered macrophages were configured to constitutively secrete TNF-$\alpha$, and TNF-$\alpha$ secretion in the liver, hepatomegaly, and increased blood AST levels were observed (see Figure 10D), as well as immune cell infiltration into the liver (see Figure 10E).

In Figures 7A and 7B, MacTrigger was administered on day 5 after tumor implantation, but in order to evaluate the effect of MacTrigger administration after the tumor had grown larger, the same dose of MacTrigger was administered on day 10 after tumor implantation. The tumor volume on day 10 was approximately twice that on day 5. The results are shown in Figure 11A. As shown in Figure 11A, MacTrigger administration exhibited a significant antitumor effect. Since the M1/M2 ratio in the tumor tissue exceeded 1 in the MacTrigger-administered group (see Figure 11A), it was considered that MacTrigger converted the tumor microenvironment from an anti-inflammatory to an inflammatory state, thereby exerting an antitumor effect.

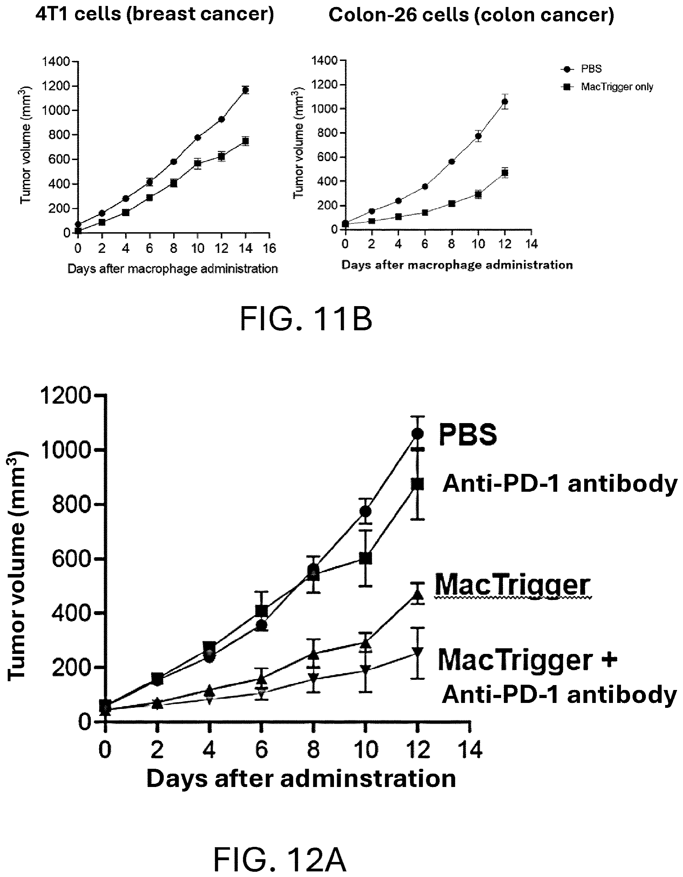

MacTrigger was administered to mice grafted with 4T1 cells, a triple-negative breast cancer cell line, or Colon-26 cells, a colon cancer cell line. As a result, a significant antitumor effect was observed in the MacTrigger-treated group compared to the negative control (PBS-treated group) (see Figure 11B).

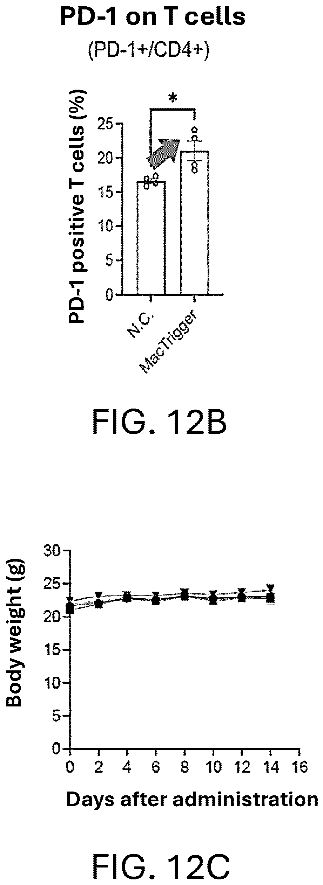

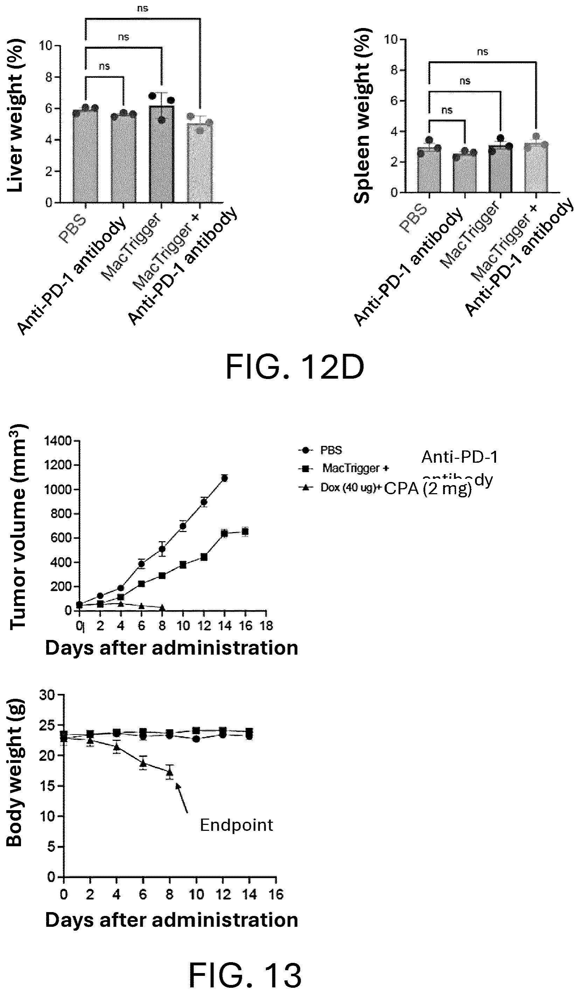

A combination with an immune checkpoint inhibitor was attempted. As the immune checkpoint inhibitor, an anti-PD-1 antibody (InVivoMAb anti-mouse PD-1, BioXcell, BE0146) was used. 4T1 cells are PD-L1 low-expressing cells and are non-responsive to treatment with anti-PD-1 antibody. A triple-negative breast cancer-bearing model was established by i.d. administration of $2 \times 10^5$ 4T1 cells into BALB/c mice (female, n = 5). 5 days later, the following treatments were intravenously administered: (i) PBS, (ii) anti-PD-1 antibody (50 $\mu$g), (iii) MacTrigger ($10^6$ cells), or (iv) a combination of anti-PD-1 antibody (50 $\mu$g) and MacTrigger ($10^6$ cells). Tumor volume and body weight were measured every other day. As shown in Figure 12A, the anti-PD-1 antibody-treated group showed little effect, as expected. In contrast, a significant antitumor effect was observed in the MacTrigger-treated group (see Figure 12A). Furthermore, in the group treated with the combination of anti-PD-1 antibody and MacTrigger, a significantly enhanced antitumor effect was observed compared to the MacTrigger-treated group (see Figure 12A). The proportion of CD4 single-positive / PD-1-positive T cells among T cells in the tumor tissue was examined. As a result, an increase in the proportion of CD4 single-positive / PD-1-positive T cells was observed in the MacTrigger-treated group (see Figure 12B). These results indicate that immune cell recruitment to the tumor is promoted; however, the proportion of immune cells expressing immune checkpoint molecules was also increased. Notably, none of the treatment groups exhibited significant body weight loss (see Figure 12C), and no other toxicities were observed (see Figure 12D). Although anti-PD-1 antibody alone was ineffective, it was unexpected that the group treated with the combination of anti-PD-1 antibody and MacTrigger showed a significantly greater antitumor effect compared to the MacTrigger-treated group. It is considered that MacTrigger transformed the tumor into an immunologically active tumor in a tumor-specific manner and induced recruitment or increase of PD-1-positive T cells into the tumor tissue that was originally non-responsive to anti-PD-1 antibody, thereby converting the tumor microenvironment into one where anti-PD-1 antibody could exert its effect.

Further experiments were conducted using a triple-negative breast cancer-bearing model. On day 5 after 4T1 cell injection, PBS was administered to the negative control group; a combination of 40 $\mu$g doxorubicin and 2 mg cyclophosphamide was administered to the comparative control group; and a combination of anti-PD-1 antibody (50 $\mu$g) and MacTrigger ($1 \times 10^6$ cells) was administered intravenously to the test group. The results are shown in Figure 13. As shown in Figure 13, although the comparative control group exhibited the strongest antitumor effect, severe side effects such as bloody stool, hematuria, and weight loss were observed, and the test was discontinued in the comparative control group. In contrast, in the test group, a significant antitumor effect was achieved without any observable side effects.

Human-type MacTrigger was prepared. From the single-cell RNA sequencing data, genes specifically expressed in M2 macrophages among M0, M1, and M2 macrophages were extracted. Sixty-one genes showed M2-specific expression. Volcano plot analysis was performed to extract genes with small p-values and large fold changes. The extracted gene expression fold changes (log2(FC)) and p-values are shown in Table 4.

[Table 4]

| Table 4: M2 expression/M0 expression ratio for each candidate gene | | | | | |
|---|---|---|---|---|---|
| Gene | log2(FC) | Adj.Pval | Gene | log2(FC) | Adj.Pval |
| FCER1A | 6.24 | 8.26E-12 | MMP1 | 2.96 | 4.13E-09 |
| F13A1 | 4.64 | 5.16E-11 | ARRDC4 | 2.93 | 3.79E-10 |
| ALDH1A2 | 4.58 | 1.84E-12 | AQP9 | 2.88 | 4.25E-09 |
| CD209 | 4.49 | 1.08E-11 | ZNF366 | 2.84 | 2.67E-10 |
| TGM2 | 4.36 | 1.08E-11 | CCL5 | 2.79 | 1.02E-09 |
| VCAM1 | 3.90 | 9.22E-12 | SOCS1 | 2.78 | 1.61E-08 |
| LOX | 3.86 | 3.66E-11 | IL1RN | 2.78 | 3.56E-09 |
| CISH | 3.68 | 9.91E-11 | IL1R1 | 2.69 | 1.18E-09 |
| ALOX15 | 3.62 | 1.39E-10 | CLEC19A | 2.66 | 3.20E-09 |
| MMP12 | 3.56 | 5.66E-09 | FABP4 | 2.62 | 6.53E-08 |
| SPINT2 | 3.55 | 3.66E-11 | GLUD1P3 | 2.57 | 4.64E-07 |
| FGL2 | 3.21 | 1.74E-10 | DUSP8P5 | 2.57 | 6.10E-09 |
| SCIMP | 3.14 | 5.60E-10 | IGFBP5 | 2.52 | 9.91E-11 |
| SIGLEC10 | 3.07 | 9.91E-11 | PPFIBP1 | 2.51 | 4.35E-09 |
| IL4I1 | 3.05 | 5.60E-10 | CD274 | 2.48 | 8.71E-09 |
| FN1 | 3.02 | 4.07E-10 | CCL22 | 2.46 | 9.91E-11 |
| SERPINB4 | 2.97 | 2.49E-10 | | | |

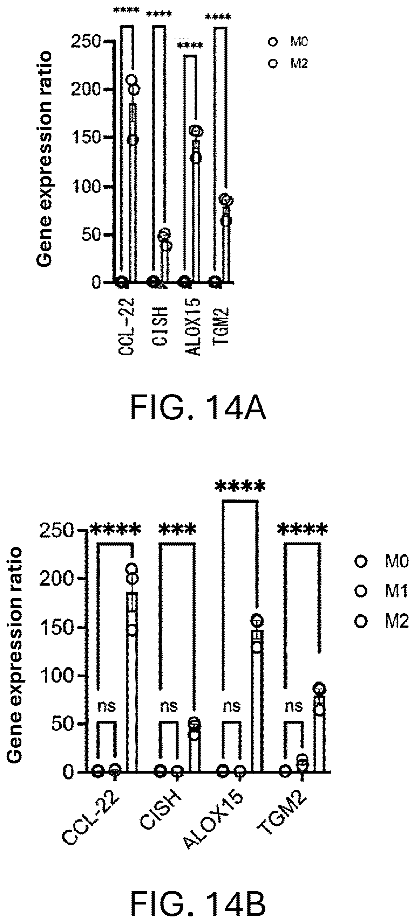

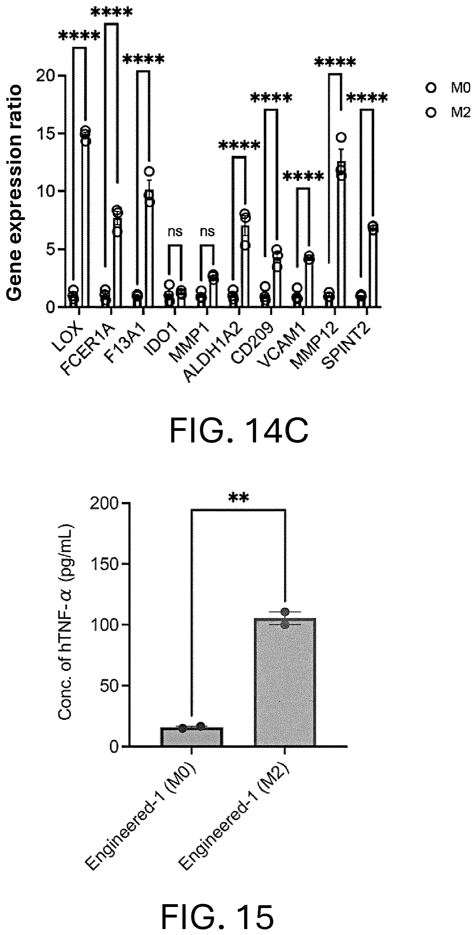

For the genes underlined in Table 3, further expression analysis was performed by RT-PCR. As shown in Figures 14A-C, most of the genes examined showed M2-specific expression. Among them, CCL-22, CISH, ALOX15, and TGM2 were revealed to be particularly preferred as promoters, as they were M2 macrophage-specific and showed high expression ratios between M2 and M0. CCL-22 refers to chemokine (C-C motif) ligand 22, CISH refers to cytokine inducible SH2-containing protein, ALOX15 refers to arachidonate 15-lipoxygenase, and TGM2 refers to transglutaminase 2.

A gene encoding human TNF-$\alpha$ was linked to the human CCL-22 promoter and introduced into THP-1 cells, and macrophages were induced by PMA treatment (20 ng/mL for 16 hours) of the THP-1 cells into which the gene had been introduced. M2 of human TNF-$\alpha$ was induced in medium containing 25 ng/mL IL-4 or IL-13. Expression levels of human TNF-$\alpha$ in M2 and M0 macrophages were measured by ELISA, and M2-specific expression of TNF-$\alpha$ was confirmed (see Figure 15).

MacTrigger is a novel type of cell-based pharmaceutical that functions as a trigger to treat cancer by inducing the immune system into tumor tissue. In this example, we report "MacTrigger," which functions as a trigger to induce an inflammatory environment exclusively in tumor tissue by modifying macrophages. As a result, strong antitumor effects based on foreign-body clearance capacity were obtained. The strength of this study lies in the utilization of two unique properties of macrophages: (1) their ability to migrate into tumor tissues with an immunosuppressive environment, and (2) their ability to polarize into an anti-inflammatory M2 phenotype in the presence of such tumor tissue. MacTrigger, when polarized to the M2 phenotype, promoted the release of the inflammatory cytokine tumor necrosis factor-alpha (TNF-$\alpha$). When MacTrigger was administered to tumor-bearing mice, tumor growth was significantly suppressed compared to untreated groups, macrophage-unmodified groups, and groups with artificial macrophages capable of randomly releasing TNF-$\alpha$. Furthermore, the M1/M2 ratio in tumor tissue exceeded 1 only in the MacTrigger group. Additionally, the proportion of natural killer cells and CD8$^+$ T cells in the tumor tissue was higher in the MacTrigger-administered group compared to the other groups. These results indicate that MacTrigger induces inflammation in tumor tissue and achieves effective antitumor effects. Moreover, no significant side effects were observed in normal tissues, particularly the liver. This is thought to be because MacTrigger did not polarize to the M2 phenotype in the liver and thus failed to induce inflammation. These results suggest that MacTrigger induces inflammation exclusively in tumor tissue and enables the immune system to attack tumor tissue via innate immunity, serving as a "trigger."

All references cited in this specification are incorporated herein by reference in their entirety.

References

[1] J. Fu et. al., J Control Release 204 (2015) 11-19.

[2] P. G. Rose et. al., Oncologist 10 (2005) 205-214.

[3] L. Yang et. al., ACS Nano 16 (2022) 9799-9809.

[4] B. Mansoori et. al., Adv Pharm Bull 7 (2017) 339-348.

[5] D. W. Edwardson et. al., Curr Drug Metab. 16 (2015) 412-426.

[6] M. Markman et. al., J. Cancer Res. Clin. Oncol. 121 (1995) 631-632.

[7] A. H. M. de Vries Schultink et. al., Eur J Clin Pharmacol 72 (2016) 645-653.

[8] W. Zou, Nat Rev Cancer 5 (2005) 263-274.

[9] T. L. Whiteside, Oncogene 27 (2008) 5904-5912.

[10] T. Wu, Y. Dai, Cancer Lett. 387 (2017) 61-68.

[11] D. H. Munn, V. Bronte, Curr Opin Immunol. 39 (2016) 1-6.

[12] T. L. Whiteside, Semin Cancer Biol 16 (2006) 3-15.

[13] A. Facciabene et. al., Cancer Res. 72 (2012) 2162-2171.

[14] R. A. Flavell et. al., Nat Rev Immunol. 10 (2010) 554-567.

[15] S. F. Josephs et. al., J Transl Med. 16 (2018). doi:10.1186/s12967-018-1611-7

[16] T. Liu et. al., Signal Transduct Target Ther. 2 (2017) 17023.

[17] S. Devaraj, I. Jialal, Arterioscler Thromb Vasc Biol. 31 (2011) 1397-1402.

[18] A. Sica et. al., Semin Cancer Biol. 18 (2008) 349-355.

[19] X. Chen et. al., Cancer Lett. 456 (2019) 69-79.

[20] W. Cai et. al., Biochem Insights. 1 (2008) 5-21.

[21] Y. Ma et. al., Sci Rep. 5 (2015) 13595.

[22] R. Gordon et. al., J Neuroinflammation 9 (2012).

[23] Y. Q. Luo et. al., IUBMB Life 58 (2006) 647-653.

[24] M. A. Lyu et. al., Biochem Pharmacol. 75 (2008) 836-846.

[25] F. Loganzo et. al., Mol Cancer Ther. 14 (2015) 952-963.

[26] C. Montagu et. al., Nat Med. 18 (2012) 221-223.

[27] L.C. Davie et. al., Nat Immunol. 14 (2013) 986-995.

[28] M. Santoni et. al., Biomed Res Int. (2014). doi:10.1155/2014/768758

[29] A. Aalipour et. al., Nat Biotechnol. 37 (2019) 531-539.

[30] C. Yunna et. al., Eur J Pharmacol. 877 (2020) 173090.

[31] P. J. Murray et. al.. Immunity, 41 (2014) 14-20.

[32] T. Roszer, Mediators Inflamm. (2015). doi:10.1155/2015/816460

[33] J. Maloney et. al., Microbes Infect. 17 (2015) 462-467.

[34] M. Munder, Br J Pharmacol. 158 (2009) 638-651.

[35] T. Veremeyko et. al., Front Immunol. 9 (2018). doi:10.3389/fimmu.2018.02515

[36] A. L. Pauleau et. al., J. Immunol. 172 (2004) 7565-7573.

[37] T. D. Schmittgen, K. J. Livak, Nat Protoc. 3 (2008) 1101-1108.

[38] R. W. Knackstedt et. al., Cancer Prevention Research. 6 (2013) 585-593.

[39] O. R. Colegio et. al., Nature 513, (2014) 559-563.

[40] C. Murdoch et. al., Blood 104 (2004) 2224-2234.

[41] M. R. McGill, EXCLI J. 15 (2016) 817-828.

[42] S. J. Lin et. al., J. Immunol 171 (2003) 4708-4716.

[43] J. Lee, S. Kim, Arch Virol 161 (2016) 1151-1158.

[44] M. M. Alvarez et. al., J Control Release. 240 (2016) 349-363.

[45] N. Wang, H. Liang Front Immunol. 5 (2014). doi:10.3389/fimmu.2014.00614

[46] S. Wang et. al., Science Translational Medicine, 13 (2021).

[47] R. Horssen et. al., Oncologist. 11 (2006) 397-408.

[48] S. Setrerrahmane, H. Xu, Mol Cancer. 16 (2017).

[49] A. Suzuki et. al., Cytokine. 75 (2015) 79-88.

[50] H. Patel et. al., Cancer Biol Ther. 19 (2018) 3-12.

[51] J. Shi et. al., Int J Mol Sci. 22 (2021) 1-16.

[52] R. Sugimoto et. al., Liver International. 25 (2005) 420-428.

[53] I. M. Wang et. al., Mol Immunol. 41 (2004) 873-884.

## Claims

1. A cell that is capable of differentiating/polarizing into an M2 macrophage, comprising a nucleic acid encoding an inflammatory cytokine operably linked to a regulatory sequence, wherein the cell is capable of expressing the inflammatory cytokine after differentiation/polarization into an M2 macrophage.

2. The cell according to Claim 1, wherein the cell is selected from the group consisting of M0 macrophages, monocytes, common monocyte progenitor cells, monocyte-dendritic progenitor cells, myeloid progenitor cells, hematopoietic stem cells, and any cell in the differentiation process from a hematopoietic stem cell to an M0 macrophage.

3. The cell according to Claim 1 or 2, wherein the cell is a macrophage that is negative for iNOS, CD206, and Arg-1.

4. The cell according to any one of Claims 1 to 3, wherein the cell is any cell in the differentiation process from a monocyte to an M0 macrophage.

5. The cell according to any one of Claims 1 to 4, wherein the inflammatory cytokine is an inflammatory cytokine selected from the group consisting of TNF-$\alpha$, IFN-$\alpha$, IFN-$\gamma$, IL-1, IL-2, IL-6, IL-8, IL-12, IL-17, and IL-23.

6. The cell according to any one of Claims 1 to 5, wherein the inflammatory cytokine is TNF-$\alpha$.

7. The cell according to any one of Claims 1 to 6, wherein the regulatory sequence drives expression of the inflammatory cytokine specifically in M2 macrophages.

8. The cell according to any one of Claims 1 to 7, wherein the regulatory sequence is a regulatory sequence of a gene selected from the group consisting of CD163, CD200R1, CD301, CXCR1, CXCR2, CD209, Dectin-1, Fc$\varepsilon$RI$\alpha$, IL-1RII, CD206, arginase 1, IRF4, PPAR$\gamma$, STAT6, FIZZ1, IL-1ra, IL-10, TGF-$\beta$, CCL1, CCL14, CCL18, CCL22, CCL23, CCL24, CCL26, and YM1.

9. A composition comprising the cell according to any one of Claims 1 to 8.

10. The composition according to Claim 9, for use in treating cancer.

A tumor tissue exhibits an immunosuppressive environment

M2 macrophage

Regulatory T cell
(Treg)

**T cell infiltration: low**

FIG. 1

**Inflammatory**

**Self-mediated therapy by the innate immune system**

FIG. 2

## FIG. 3

## FIG. 4

FIG. 5A

FIG. 5B

FIG. 5C

FIG. 6A

FIG. 6B

FIG. 6C

FIG. 7A

FIG. 7B

FIG. 7C

FIG. 7D

FIG. 7E

FIG. 8A

FIG. 8B

_In vivo_ imaging

1d          4d          7d

FIG. 9A

FIG. 9B

## Ex vivo imaging

**1d**

**4d**

**7d**

FIG. 9C

Cells from tissues

- BV421-anti-mouse F4/80
- Alexa Fluor488-anti-mouse CD11b
- PE-anti-mouse CD206

Analyzed by flow cytometry

(F4/80$^+$ & CD11b$^+$ & VT-680$^+$)

FIG. 10A

AST assay

FIG. 10B

FIG. 10C

FIG. 10D

PBS　　Mac (UTD)　　Mac (CMVp)　　MacTrigger
Infiltration of
immune cells

FIG. 10E

Days after administration

FIG. 11A

**4T1 cells (breast cancer)** **Colon-26 cells (colon cancer)**

FIG. 11B

FIG. 12A

**PD-1 on T cells**

(PD-1+/CD4+)

FIG. 12B

FIG. 12C

FIG. 12D

FIG. 13

FIG. 14A

FIG. 14B

FIG. 14C

FIG. 15

# Schematic diagram of this study

FIG. 16

## INTERNATIONAL SEARCH REPORT

| International application No. |
|---|
| **PCT/JP2024/004080** |

### A. CLASSIFICATION OF SUBJECT MATTER

*C12N 5/10*(2006.01)i; *A61K 35/15*(2015.01)i; *A61P 35/00*(2006.01)i; *C12N 15/19*(2006.01)i; *C12N 15/28*(2006.01)i; *C12N 5/0786*(2010.01)n
FI: C12N5/10 ZNA; A61K35/15; A61P35/00; C12N15/19; C12N15/28; C12N5/0786

According to International Patent Classification (IPC) or to both national classification and IPC

### B. FIELDS SEARCHED

Minimum documentation searched (classification system followed by classification symbols)

C12N5/10; A61K35/15; A61P35/00; C12N15/19; C12N15/28; C12N5/0786

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | JP 2020-517261 A (OSPEDALE SAN RAFFAELE S.R.L) 18 June 2020 (2020-06-18) claims, paragraph [0243], example 7, fig. 28-30 | 1-10 |
| A | JP 2021-126113 A (YAMAGUCHI UNIVERSITY) 02 September 2021 (2021-09-02) entire text, all drawings | 1-10 |
| A | JP 2021-514619 A (THE BOARD OF TRUSTEES OF THE LELAND STANFORD JUNIOR UNIVERSITY) 17 June 2021 (2021-06-17) entire text, all drawings | 1-10 |
| A | WO 2021/138302 A1 (UNIVERSITY OF PITTSBURGH - OF THE COMMONWEALTH SYSTEM OF HIGHER EDUCATION) 08 July 2021 (2021-07-08) entire text, all drawings | 1-10 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **03 April 2024** | **23 April 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/004080**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

a. ☑ forming part of the international application as filed.

b. ☐ furnished subsequent to the international filing date for the purposes of international search (Rule 13*ter*.1(a)),

☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/JP2024/004080**

| Patent document cited in search report | | | Publication date (day/month/year) | Patent family member(s) | | | Publication date (day/month/year) |
|---|---|---|---|---|---|---|---|
| JP | 2020-517261 | A | 18 June 2020 | US | 2020/0078408 | A1 | |
| | | | | claims, paragraph [0316], example 7, fig. 28-30 | | | |
| | | | | EP | 3612624 | A1 | |
| | | | | WO | 2018/193119 | A1 | |
| | | | | KR | 10-2020-0020677 | A | |
| | | | | CN | 110785488 | A | |
| JP | 2021-126113 | A | 02 September 2021 | WO | 2021/161997 | A1 | |
| JP | 2021-514619 | A | 17 June 2021 | US | 2021/0011006 | A1 | |
| | | | | entire text, all drawings | | | |
| | | | | EP | 3759494 | A1 | |
| | | | | WO | 2019/168948 | A1 | |
| | | | | CN | 111868234 | A | |
| WO | 2021/138302 | A1 | 08 July 2021 | US | 2023/0058380 | A1 | |
| | | | | EP | 4084807 | A1 | |
| | | | | JP | 2023-508498 | A | |
| | | | | KR | 10-2022-0122636 | A | |
| | | | | CN | 115443141 | A | |

Form PCT/ISA/210 (patent family annex) (July 2022)

EP 4 663 750 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 92063934 B

### Non-patent literature cited in the description

- **J. FU**. *J Control Release*, 2015, vol. 204, 11-19
- **P. G. ROSE**. *Oncologist*, 2005, vol. 10, 205-214
- **L. YANG**. *ACS Nano*, 2022, vol. 16, 9799-9809
- **B. MANSOORI**. *Adv Pharm Bull*, 2017, vol. 7, 339-348
- **D. W. EDWARDSON**. *Curr Drug Metab.*, 2015, vol. 16, 412-426
- **M. MARKMAN**. *J. Cancer Res. Clin. Oncol.*, 1995, vol. 121, 631-632
- **A. H. M. DE VRIES SCHULTINK**. *Eur J Clin Pharmacol*, 2016, vol. 72, 645-653
- **W. ZOU**. *Nat Rev Cancer*, 2005, vol. 5, 263-274
- **T. L. WHITESIDE**. *Oncogene*, 2008, vol. 27, 5904-5912
- **T. WU ; Y. DAI**. *Cancer Lett.*, 2017, vol. 387, 61-68
- **D. H. MUNN ; V. BRONTE**. *Curr Opin Immunol.*, 2016, vol. 39, 1-6
- **T. L. WHITESIDE**. *Semin Cancer Biol*, 2006, vol. 16, 3-15
- **A. FACCIABENE**. *Cancer Res.*, 2012, vol. 72, 2162-2171
- **R. A. FLAVELL**. *Nat Rev Immunol.*, 2010, vol. 10, 554-567
- **S. F. JOSEPHS**. *J Transl Med.*, 2018, 16
- **T. LIU**. *Signal Transduct Target Ther.*, 2017, vol. 2, 17023
- **S. DEVARAJ ; I. JIALAL**. *Arterioscler Thromb Vasc Biol.*, 2011, vol. 31, 1397-1402
- **A. SICA**. *Semin Cancer Biol.*, 2008, vol. 18, 349-355
- **X. CHEN**. *Cancer Lett.*, 2019, vol. 456, 69-79
- **W. CAI**. *Biochem Insights.*, 2008, vol. 1, 5-21
- **Y. MA**. *Sci Rep.*, 2015, vol. 5, 13595
- **R. GORDON**. *J Neuroinflammation*, 2012, 9
- **Y. Q. LUO**. *IUBMB Life*, 2006, vol. 58, 647-653
- **M. A. LYU**. *Biochem Pharmacol.*, 2008, vol. 75, 836-846
- **F. LOGANZO**. *Mol Cancer Ther.*, 2015, vol. 14, 952-963
- **C. MONTAGU**. *Nat Med.*, 2012, vol. 18, 221-223
- **L.C. DAVIE**. *Nat Immunol.*, 2013, vol. 14, 986-995
- **M. SANTONI**. *Biomed Res Int.*, 2014
- **A. AALIPOUR**. *Nat Biotechnol.*, 2019, vol. 37, 531-539
- **C. YUNNA**. *Eur J Pharmacol.*, 2020, vol. 877, 173090
- **P. J. MURRAY**. *Immunity*, 2014, vol. 41, 14-20
- **T. ROSZER**. *Mediators Inflamm.*, 2015
- **J. MALONEY**. *Microbes Infect.*, 2015, vol. 17, 462-467
- **M. MUNDER**. *Br J Pharmacol.*, 2009, vol. 158, 638-651
- **T. VEREMEYKO**. *Front Immunol.*, 2018, 9
- **A. L. PAULEAU**. *J. Immunol.*, 2004, vol. 172, 7565-7573
- **T. D. SCHMITTGEN ; K. J. LIVAK**. *Nat Protoc.*, 2008, vol. 3, 1101-1108
- **R. W. KNACKSTEDT**. *Cancer Prevention Research.*, 2013, vol. 6, 585-593
- **O. R. COLEGIO**. *Nature*, 2014, vol. 513, 559-563
- **C. MURDOCH**. *Blood*, 2004, vol. 104, 2224-2234
- **M. R. MCGILL**. *EXCLI J.*, 2016, vol. 15, 817-828
- **S. J. LIN**. *J. Immunol*, 2003, vol. 171, 4708-4716
- **J. LEE ; S. KIM**. *Arch Virol*, 2016, vol. 161, 1151-1158
- **M. M. ALVAREZ**. *J Control Release.*, 2016, vol. 240, 349-363
- **N. WANG ; H. LIANG**. *Front Immunol.*, 2014, 5
- **S. WANG**. *Science Translational Medicine*, 2021, 13
- **R. HORSSEN**. *Oncologist.*, 2006, vol. 11, 397-408
- **S. SETRERRAHMANE ; H. XU**. *Mol Cancer.*, 2017, 16
- **A. SUZUKI**. *Cytokine*, 2015, vol. 75, 79-88
- **H. PATEL**. *Cancer Biol Ther.*, 2018, vol. 19, 3-12

- **J. SHI**. *Int J Mol Sci.*, 2021, vol. 22, 1-16
- **R. SUGIMOTO**. *Liver International.*, 2005, vol. 25, 420-428
- **I. M. WANG**. *Mol Immunol.*, 2004, vol. 41, 873-884